# EUROPEAN PATENT SPECIFICATION

(11) **EP 2 182 961 B1**
(45) Date of publication and mention of the grant of the patent: **02.05.2012**
(21) Application number: 08788928.3
(22) Date of filing: 24.07.2008
(51) Int. Cl.: A61K 33/00, A61P 1/04

(54) **PREVENTION OF GASTRIC ULCERY BY CARBON MONOXIDE**
VERHINDERUNG VON MAGENGESCHWÜREN MIT KOHLENMONOXID
PRÉVENTION D'UN ULCÈRE GASTRIQUE PAR LE MONOXYDE DE CARBONE

(30) Priority: 24.07.2007 US 951650 P
(43) Date of publication of application: 12.05.2010
(73) Proprietor: Alfama - Investigaçao e Desenvolvimento de Productos Farmaceuticos LDA., 2740-122 Porto Salvo (PT)
(72) Inventor: RODRIGUES, Sandra, S., P-1990-177 Lisboa (PT); SEIXAS, João, D., P-2675-394 Odivelas (PT); GUERREIRO, Bruno, P-2680-409 Unhos (PT); PEREIRA, Nuno, Miguel, Penacho, P-1500-244 Lisboa (PT); ROMÃO, Carlos, C., P-2750-768 Cascais (PT); HAAS, Werner, E., P-2780 Oeiras (PT); GONÇALVES, Isabel, Maria de Sousa, P-3800-171 Aveiro (PT)
(74) Representative: Paget, Hugh Charles Edward
(86) International application number: PCT/IB2008/001945
(87) International publication number: WO 2009/013612

(56) References cited:
- WO-A-03/066067
- WO-A-2008/130261
- MOTTERLINI R ET AL: "Therapeutic applications of carbon monoxide-releasing molecules" EXPERT OPINION ON INVESTIGATIONAL DRUGS, ASHLEY PUBLICATIONS LTD., LONDON, GB, vol. 14, no. 11, 1 January 2005 (2005-01-01), pages 1305-1318, XP007905649 ISSN: 1354-3784

## Description

### BACKGROUND OF THE INVENTION

Aspirin and other nonsteroidal anti-inflammatory drugs (NSAIDs) are widely used as analgesic, anti-inflammatory and anti-pyretic drugs. These drugs are generally well tolerated. However, gastrointestinal side effects are common. At least every second NSAIDs user suffers from epigastric pain (dyspepsia) and/or has erosions of the intestinal mucosa, in particular in the stomach and the duodenum. Severe, harmful gastrointestinal events such as bleeding, perforation or obstruction occur in about 1 of 100 NSAID users (for review see Laine 1996; Wolfe, Lichtenstein et al. 1999; Hawkey 2000).

Only a few of many efforts have succeeded to reduce NSAID-induced dyspepsia and/or the risk of harmful events (for review see (Rostom, Dube et al. 2002; Hooper, Brown et al. 2004). The gastrointestinal toxicity of NSAIDs can be reduced by coadministration of the stable prostaglandin derivative misoprostol (Silverstein 1998), a proton pump inhibitor such as omeprazole (Ekstrom, Carling et al. 1996), and, to a lesser extent, by coadminsistration of a histamine H2 receptor antagonists such as ranitidine (Zantac). Misoprostol inhibits acid secretion, enhances secretion of mucus and bicarbonate and improves mucosal blood flow. However, misoprostol does not reduce NSAID associated dyspepsia, requires multiple daily doses and has side effects such as diarrhea often associated with abdominal pain and cramps (Raskin, White et al. 1995). While proton pump inhibitors do reduce the incidence of NSAID ulcers in patients with a history of uncomplicated gastric ulcers, they do not affect clinically important outcomes, namely mortality, rebleeding or need for surgery (Dorward, Sreedharan et al. 2006).

Some physicians believe that coadministration of protective agents such as misoprostol and proton pump inhibitors are under-utilized by NSAID users (Goldstein 2004). Likely reasons for this are the additional costs, side effects of the supplementary drugs themselves, and the need for frequent dosing in the case of misoprostol. Moreover, patients as well as many physicians tend to underestimate the threat of serious side-effects, since NSAIDs do not cause such harmful events in the majority of NSAID users.

The ideal solution for the "NSAID problem" would be NSAIDs with reduced gastrointestinal side effects. The chance to find such compounds appeared to be low, since cyclooxygenase (COX) inhibition was responsible for both beneficial and adverse effects and none of the many NSAIDs developed in the second half of the last century appeared to be free of gastrointestinal toxicity. However, research on NSAIDs dramatically changed with the discovery of a second cyclooxygenase isoform (COX-2) and the development of selective COX-2 inhibitors, now frequently referred to as COXIBs (Needleman and Isakson 1997; Flower 2003). Initial research on the two COX isoforms in health and disease suggested that the beneficial effects of NSAIDs were due to inhibition of the inducible isoform, COX-2, while the adverse effects in the gastrointestinal tract were attributed to the inhibition of the constitutively expressed isoform, COX-1. Although this view is now known to be an oversimplification, COXIBs had the expected properties. While they were as effective as traditional NSAIDs in reducing pain and inflammation, they were clearly less toxic to the gastrointestinal tract in animals and humans (McMurray and Hardy 2002; de Leval, Julemont et al. 2004). COXIBs rapidly became multi-billion dollar drugs. Besides the use of COXIBs for the treatment of pain due to inflammatory diseases and other causes, new indications were considered, in particular Alzheimer's disease (McGeer 2000), prevention of colon cancer in patients with a predisposition (Prescott and Fitzpatrick 2000; Jacobs, Thun et al. 2007) and conditions associated with pathological angiogenesis (Jones, Wang et al. 1999).

Since COXIBs spare COX-1, they do not inhibit thrombosis, and thus do not have the cardioprotective effects that are well established for aspirin. Moreover, clinical trials with COXIBs showed that these novel NSAIDs were not only not cardioprotective, but increased the risk of cardiovascular events (Howard and Delafontaine 2004; Juni, Nartey et al. 2004) This finding resulted in the withdrawal of rofecoxib (Vioxx) and of other COXIBs from the market (Topol 2004). At the present time there is much uncertainty with regard to the ratio of beneficial and adverse effects of different NSAIDs in general and of COXIBs in particular (Rahme and Nedjar 2007; Rostom, Muir et al. 2007; Salinas, Rangasetty et al. 2007).

Another strategy to increase the safety of NSAIDs has been to attach a nitric oxide (NO) releasing moiety to NSAIDs. With the realization that COXIBs were not the final perfection of NSAIDs, NO-NSAIDs received more attention. Promising results were obtained with these compounds in animal experiments. NO-NSAIDs had less gastrointestinal side effects and the same or even improved beneficial effects as compared to the parent NSAIDs (for review see Bandarage and Janero 2001; Keeble and Moore 2002; Fiorucci and Antonelli 2006). The results of preclinical studies were confirmed in early clinical trials. Endoscopic examinations revealed less gastroduodenal erosions and hemorrhagic lesions in volunteers or patients who received NO-aspirin (Fiorucci, Santucci et al. 2003) or NO-naproxen (Wilder-Smith, Jonzon et al. 2006) as compared to persons who received equimolar doses of aspirin and naproxen, respectively. Based on these findings it is expected that NO-NSAIDs use will result in less harmful events (i.e. bleeding or perforation) than traditional NSAID.

At the present time NO-naproxen and NO-aspirin are in advanced clinical development for the treatment of patients with osteoarthritis and patients with type 2 diabetes, respectively (Fiorucci and Antonelli 2006). A variety of clinical NO-NSAID applications are claimed in several patents (WO1995009831; US5700947; US5621000; US5861426; US6040341; US6242432 B1). NO generated from NO-NSAIDs after enzymatic processing is thought to protect the gastric mucosa against NSAID mediated damage. NO increases blood flow in the gastric microcirculation, inhibits leukocyte adherence to endothelial cells, inhibits apoptosis of endothelial and epithelial cells and increases mucus secretion (Elliott and Wallace 1998).

There are two potential drawbacks of the NO-NSAID strategy. First, since the NO donating group is the same as that in organic nitrates, it is likely that treatment of NO-NSAIDs will lead to a refractory time period of about 8 hours during which both endogenous and exogenous NO remain ineffective, a phenomenon known as nitrate tolerance (Miller and Megson 2007; Minamiyama, Takemura et al. 2007). Indeed, cross-tolerance between NO-aspirin and nitroglycerin has been observed (Grosser and Schroder 2000). Second, the NO generated from NO donor drugs such as nitroglycerin and nitroprusside is known to be mutagenic (Birnboim and Privora 2000). While the mutagenicity of NO may be of little relevance in the cardiovascular applications of these drugs, it may preclude the long term use of NO-NSAIDs in the treatment of chronic diseases such as arthritis and diabetes.

Carbon monoxide (CO), like NO, is an important biological signalling molecule. Most of the carbon monoxide that is generated in the human body derives from the oxidation of heme by heme oxygenases that occur in two isoforms. One isoform (HO-2) is constitutively expressed in specific tissues such as the vasculature and the brain; the other isoform (HO-1) is induced by a wide variety of endogenous and exogenous stimuli (for review see (Maines 1997). CO produced by these enzymes has vasodilatory, anticoagulant, antiinflammatory and neurotransmitter functions. In animal experiments, inhalation of CO at low doses has beneficial effects in a wide variety of pathological conditions, most of which are associated with inflammation and ischemia reperfusion injury (Ryter and Otterbein 2004). Similar beneficial effects have been achieved by the administration of CO releasing molecules also known as CORMs (for review see Motterlini, Mann et al. 2003). The cellular and molecular mechanisms underlying the effects of endogenously produced CO and of therapeutically administered CO are poorly understood, mainly because of a complex network of signals involving CO and other mediators, and because of the difficulty to accurately measure CO levels in cells and tissues.

### SUMMARY OF THE INVENTION

The present invention discloses the use of carbon monoxide (CO) to prevent the gastrointestinal side effects of NSAIDs.

According to an aspect of the invention, a use of CO is claimed. The use is of CO in the manufacture of a medicament for the treatment of NSAID or alcohol induced gastrointestinal side-effects, e.g. gastric ulcer. In one important embodiment the CO is in the form of a dissolved gas, which may or may not be trapped in a carrier complex. In another important embodiment, the CO is in the form of a prodrug, such as a CORM. In important embodiments, the CORM is a CO containing organometallic complex or an organic compound. Numerous CORMs are described herein and are suitable for the practice of the present invention.

According to another aspect of the invention, CO for use in, a method is provided. The method is one for inhibiting NSAID or alcohol induced gastrointestinal side-effects, e.g. gastric ulcer. The method involves administering to a subject in need of such inhibition an effective amount of CO. In one important embodiment, the subject is undergoing NSAID treatment for inflammation, pain, fever, a thromboembolic disorder, patent ductus arteriosus, Bartter's syndrome, cancer, a condition associated with pathological angiogenesis, psoriasis, Alzheimer's disease, and/or shock. In one important embodiment, the subject is undergoing NSAID treatment for inflammation. In one important embodiment, the subject is undergoing NSAID treatment for pain. In one important embodiment, the subject is undergoing NSAID treatment for a condition other than an inflammatory condition.

In any of the forgoing embodiments of this aspect of the invention, the CO can be administered as a gas or as a CORM. In one important embodiment, the CORM is a CO containing organometallic complex. In one important embodiment, the CORM is an organic compound. The CORM can be formulated in any pharmaceutically acceptable manner. In one preferred embodiment, the CORM (or the gas) is delivered locally, such as orally to the stomach. In one embodiment, the CORM is formulated with a carrier that binds to the mucosal surface in the stomach and/or the duodenum. In one embodiment, the CORM is delivered in an alginate solution.

In any of the forgoing embodiments of this aspect of the invention, the method can further comprise administering an NSAID to the subject. The NSAID may be administered separately from the CORM or the gas, or the NSAID can be admixed with the CORM or the gas. In one embodiment, the NSAID is covalently conjugated to the CORM, preferably via a digestible linker.

According to another aspect of the invention, CO for use in method is provided to inhibit NSAID or alcohol induced gastrointestinal side effects, e.g., gastric ulcer. The method involves instructing a subject undergoing NSAID therapy to take an effective amount of CO for the purpose of inhibiting NSAID or alcohol induced gastric ulcer. In one important embodiment, the subject is undergoing NSAID treatment for inflammation, pain, fever, a thromboembolic disorder, patent ductus arteriosus, Bartter's syndrome, cancer, a condition associated with pathological angiogenesis, psoriasis, Alzheimer's disease, and/or shock. In one important embodiment, the subject is undergoing NSAID treatment for inflammation. In one important embodiment, the subject is undergoing NSAID treatment for pain. In one important embodiment, the subject is undergoing NSAID treatment for a condition other than an inflammatory condition.

In any of the forgoing embodiments of this aspect of the invention, the patient can be instructed to take CO as a gas or as a CORM. In one important embodiment, the CORM is a CO containing organometallic complex. In one important embodiment, the CORM is an organic compound. The CORM can be formulated in any pharmaceutically acceptable manner. In one preferred embodiment, the CORM (or the gas) is delivered locally, such as orally to the stomach. In one embodiment, the CORM is formulated with a carrier that binds to the mucosal surface in the stomach and/or the duodenum. In one embodiment, the CORM is delivered in an alginate solution.

In any of the forgoing embodiments of this aspect of the invention, the method can further comprise instructing the patient to take an NSAID. The NSAID may be administered separately from the CORM or the gas, or the NSAID can be admixed with the CORM or the gas. In one embodiment, the NSAID is covalently conjugated to the CORM, preferably via a digestible linker.

According to another aspect of the invention, CO for use in a method is provided to treat a subject to inhibit NSAID or alcohol induced gastrointestinal side-effects, e.g. gastric ulcer. The method involves providing the subject with a package containing a CORM, and providing the subject with indicia indicating that the CORM is for inhibiting NSAID or alcohol induced gastric ulcer. In one important embodiment, the subject is undergoing NSAID treatment for inflammation, pain, fever, a thromboembolic disorder, patent ductus arteriosus, Bartter's syndrome, cancer, a condition associated with pathological angiogenesis, psoriasis, Alzheimer's disease, and/or shock. In one important embodiment, the subject is undergoing NSAID treatment for inflammation. In one important embodiment, the subject is undergoing NSAID treatment for pain. In one important embodiment, the subject is undergoing NSAID treatment for a condition other than an inflammatory condition.

In any of the forgoing embodiments of this aspect of the invention, the CORM can be a CO containing organometallic complex or an organic compound. The CORM can be formulated in any pharmaceutically acceptable manner. In one preferred embodiment, the CORM is formulated to be delivered locally, such as orally to the stomach. In one embodiment, the CORM is formulated with a carrier that binds to the mucosal surface in the stomach and/or the duodenum. In one embodiment, the CORM is formulated in an alginate solution.

In any of the forgoing embodiments of this aspect of the invention, the method can further comprise providing the patient with an NSAID or instructing the patient to take an NSAID. The patient may be provided with indicia indicating the the NSAID is for inflammation, pain, fever, a thromboembolic disorder, patent ductus arteriosus, Bartter's syndrome, cancer, a condition associated with pathological angiogenesis, psoriasis, Alzheimer's disease, and/or shock. In one aspect, the indicia indicates that the NSAID is for inflammation or for a condition other than an inflammatory condition, such as for pain. The NSAID may be administered separately from the CORM or the gas, or the NSAID can be admixed with the CORM or the gas. In one embodiment, the NSAID is covalently conjugated to the CORM, preferably via a digestible linker.

Also disclosed is a medical treatment product is provided. The product is a package containing a CORM and containing indicia indicating that the CORM is for inhibiting NSAID or alcohol induced gastric ulcer. In one aspect, the CORM is in a bottle. In a further aspect, the indicia is on a label on the bottle.

In any of the forgoing embodiments of this aspect of the invention, the CORM can be a CO containing organometallic complex or an organic compound. The CORM can be formulated in any pharmaceutically acceptable manner. In one preferred embodiment, the CORM is formulated to be delivered locally, such as orally to the stomach. In one embodiment, the CORM is formulated with a carrier that binds to the mucosal surface in the stomach and/or the duodenum. In one embodiment, the CORM is formulated in an alginate solution.

In any of the forgoing embodiments of this aspect of the invention, the package further can contain an NSAID and optionally indicia instructing the patient to take the NSAID. In one aspect, the NSAID is for inflammation, pain, fever, a thromboembolic disorder, patent ductus arteriosus, Bartter's syndrome, cancer, a condition associated with pathological angiogenesis, psoriasis, Alzheimer's disease, another aspect and/or shock. In another aspect, the indicia indicates that the NSAID is for inflammation or for a condition other than an inflammatory condition, such as for pain. The NSAID may be in a container separate from the CORM or the NSAID can be admixed with the CORM in the same container. In one embodiment, the NSAID is covalently conjugated to the CORM, preferably via a digestible linker, e.g. an ester bond.

It will be understood, that in some embodiments, the CO is generated within the gastrointestinal tract from an orally administered prodrug such as a CORM. The prodrug can be targeted to various places in the gastrointestinal tract, such as the upper gastrointestinal tract by an appropriate formulation. The prodrug also can be bound to a targeting molecule that binds to the mucosal surface in the stomach and the duodenum.

Also disclosed is a method for inhibiting NSAID or alcohol induced gastrointestinal side-effects, e.g. gastric ulcer. The method involves administering to a subject in need of such inhibition an effective amount of an agent that increases endogenous carbon monoxide production by inducing the expression of heme oxygenase 1. In one aspect, the agent is administered by the oral route, preferably targeted to the upper gastrointestinal tract. The agent can be formulated with or attached to an agent that targets the agent to the upper gastrointestinal tract.

In any of the foregoing embodiments, the NSAID which induces the gastrointestinal side-effect can be any such NSAID causing the same. Important NSAIDs are listed below. In particularly important embodiments, the NSAID is aspirin, a nonselective cyclooxygenase inhibitor including but not limited to ibuprofen, naproxen, flurbiprofen, indomethacin, diclofenac, etodolac, piroxicam, meloxicam, tenoxicam, nimesulide, or a selective cyclooxygenase 2 inhibitor including but not limited to celcoxib, rofecoxib, and valdecoxib.

In any of the foregoing embodiments, the gastrointestinal side effect include but are not limited to dyspepsia, gastroesophageal reflux, gastrointestinal bleeding, perforation and obstruction.

### BRIEF DESCRIPTION OF THE FIGURES AND TABLES

Figure 1: Grading the severity of stomach ulcers according to a score between 0 and 3. Three independent examples for each score are given in each column.
Figure 2: Systemic administration of a CORM (ALF186 (Formula I)) is less effective than oral administration in protecting rats against indomethacin induced ulcers
Table 1: Protection against ethanol induced ulcers by a CORM (ALF 186) in rats
Table 2: Protection against indomethacin induced ulcers by a CORM (ALF186) in rats
Table 3: The CORM ALF186 does not interfere with the anti-inflammatory effect of indomethacin in rats.
Table 4: Systemic administration of a CORM (ALF186) is less effective than oral administration in protecting rats against indomethacin induced ulcers
Table 5: Systemic administration of a CORM (ALF186) is less effective than oral administration in protecting rats against indomethacin induced ulcers

### DETAILED DESCRIPTION OF THE INVENTION

To our surprise, we found that the induction of gastric ulcers by ethanol or NSAIDs could be prevented by the administration of CO.

NSAIDs of all types can cause gastric ulcer in subjects. NSAIDs are well known and include: salicylic acid derivatives (such as salicylic acid, acetylsalicylic acid, glycol salicylate, methyl salicylate, diflunisal, olsalazine, salsalate and sulfasalazine), indole and indene acetic acids (such as indomethacin, etodolac and sulindac), fenamates (such as etofenamic, meclofenamic, mefenamic, flufenamic, niflumic and tolfenamic acids), heteroaryl acetic acids (such as acemetacin, alclofenac, amfenac, bromfenac, clidanac, diclofenac, fenchlofenac, fentiazac, furofenac, ibufenac, isoxepac, ketorolac, oxipinac, tiopinac, tolmetin, zidometacin and zomepirac), aryl acetic acid and propionic acid derivatives (such as alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid and tioxaprofen), enolic acids (such as the oxicam derivatives ampiroxicam, cinnoxicam, droxicam, lornoxicam, meloxicam, piroxicam, sudoxicam and tenoxicam, and the pyrazolone derivatives aminopyrine, antipyrine, apazone, dipyrone, oxyphenbutazone and phenylbutazone), alkanones (such as nabumetone), nimesulide, proquazone, MX-1094, licofelone, 3-amino-4-hydroxybutyric acid, bromosaligenin, bumadizon, ditazol, enfenamic acid, etofenamate, fendosal, fepradinol, flufenamic acid, gentisic acid, glucamethacin, meclofenamic acid, mefenamic acid, mesalamine, niflumic acid, olsalazine, oxaceprol, S-adenosylmethionine, and tolfenamic acid.

NSAIDs also include cyclooxygenase-2 (COX-2) inhibitors, such as, e.g., celecoxib, cimicoxib, deracoxib, etoricoxib, lumiracoxib, meloxicam, onconoxib, parecoxib, rofecoxib, tilmacoxib, valdecoxib, PAC-10549, GW-406381, LAS-34475, or CS-502. A number of selective COX-2 inhibitors are described in U.S. Patent Nos. 5,474,995; 5,521,213; 5,536,752; 5,550,142; 5,552,422; 5,604,253; 5,604,260; 5,639,780; 5,677,318; 5,691,374; 5,698,584; 5,710,140; 5,733,909; 5,789,413; 5,817,700; 5,849,943; 5,861,419; 5,922,742; 5,925,631. Additional COX-2 inhibitors are also described in U.S. Patent 5,643,933.

A number of the above-identified COX-2 inhibitors are prodrugs of selective COX-2 inhibitors, and exert their action by conversion *in vivo* to the active and selective COX-2 inhibitors. The active and selective COX-2 inhibitors formed from the above-identified COX-2 inhibitor prodrugs are described in detail in WO 95/00501, published January 5, 1995, WO 95/18799, published July 13, 1995 and U.S. Patent 5,474,995, issued December 12, 1995.

In important embodiments, the CO is used to inhibit gastric ulcer in subjects undergoing therapy with aspirin, a nonselective cyclooxygenase inhibitor that is ibuprofen, naproxen, flurbiprofen, indomethacin, diclofenac, etodolac, piroxicam, meloxicam, tenoxicam, and/or nimesulide, or a selective cyclooxygenase 2 inhibitor that is celcoxib, rofecoxib, and/or valdecoxib, or combinations thereof.

The present invention involved the inhibition of gastrointestinal side effects in subjects caused by NSAID therapy, including but not limited to dyspepsia, gastroesophageal reflux, gastrointestinal bleeding, perforation and obstruction. In one important embodiment, the gastrointestinal side effect is gastric ulcer.

Subjects, as used herein, means humans, nonhuman primates, dogs, cats, horses, sheep, goats, cows, pigs, and rodents. The subjects may be taking NSAIDs acutely, long term, or chronically. For example, the subjects may be taking the NSAIDs once a day or several times a day, for 1, 2, 3, 4, 5, 6, 7, or more days. The subjects may be taking the NSAIDs once a day or several times a day, for 1, 2, 3, 4, 5, 6, 7, 8 or more weeks. The subjects may be taking the NSAIDs once a day or several times a day, for 1, 2, 3, 4, 5, 6 or more months. Likewise, the subjects may be taking NSAIDs intermittently over such time periods, such as every other day, every few days, or "as needed". As used herein, "long term" means daily or intermittent use at least three times a week for at least one month and "chronic" means daily or intermittent use for at least three times a week for at least six months.

NSAIDs can be taken for a variety of conditions to achieve a variety of results. For example, NSAIDs are prescribed and used for treatment of inflammatory conditions due to their anti-inflammatory properties. As used herein, inflammatory conditions means: transplant rejection; chronic inflammatory disorders of the joints, such as arthritis, rheumatoid arthritis, juvenile idiopathic arthritis, ankylosing spondylitis, psoriatic arthritis, osteoarthritis and bone diseases associated with increased bone resorption; inflammatory bowel diseases, such as ileitis, ulcerative colitis, Barrett's syndrome, and Crohn's disease; inflammatory lung disorders, such as asthma, adult respiratory distress syndrome (ARDS), chronic obstructive pulmonary disease (COPD) or chronic obstructive airway disease; inflammatory disorders of the eye, such as corneal dystrophy, trachoma, onchocerciasis, uveitis, sympathetic ophthalmitis and endophthalmitis; chronic inflammatory disorders of the gum, such as gingivitis and periodontitis; tuberculosis; leprosy; inflammatory diseases of the kidney, such as uremic complications, glomerulonephritis and nephrosis; inflammatory diseases of the liver, such as viral hepatitis and autoimmune hepatitis; inflammatory disorders of the skin, such as sclerodermatitis, psoriasis, erythema, eczema, or contact dermatitis; inflammatory diseases of the central nervous system, such as stroke, chronic demyelinating diseases of the nervous system, multiple sclerosis, AIDS-related neurodegeneration and Alzheimer's disease, infectious meningitis, encephalomyelitis, Parkinson's disease, Huntington's disease, amyotrophic lateral sclerosis and viral or autoimmune encephalitis; autoimmune diseases, such as diabetes mellitus, immune-complex vasculitis, systemic lupus erythematosus (SLE); inflammatory diseases of the heart, such as cardiomyopathy, ischemic heart disease, hypercholesterolemia, and atherosclerosis; as well as inflammation resulting from various diseases such as preeclampsia, chronic liver failure, brain and spinal cord trauma, and cancer. Inflammatory diseases treatable as described herein further include systemic inflammations of the body. Examples of systemic inflammation include but are not limited to gram-positive or gram negative shock, sepsis, septic shock, hemorrhagic or anaphylactic shock, and systemic inflammatory response syndrome. Further examples of inflammatory disease include circulatory shock, hemorrhagic shock and cardiogenic shock.

NSAIDs also can be used to treat pain due to their analgesic properties, independent of their anti-inflammatory properties. In particular, NSAIDs can be used to treat neuropathic pain, acute pain, chronic pain, post-operative pain, acute and chronic lower back pain, headaches, neck pain, cluster headaches, period (dysmenorrhea), kidney stones (renal colic), pain resulting from sprains or strains, herpes neuralgia, phantom limb pain, central pain, dental pain, resistant pain, visceral pain, surgical pain, bone injury pain, pain during labor and delivery, pain resulting from bums, pain resulting from sunburns, post partum pains, migraine, angina pain, genitourinary tract-related pain, and/or nociceptive pain.

NSAIDs also can be used to treat fever, thromboembolic disorders, patent ductus arteriosus, Bartter's syndrome, cancer, conditions associated with pathological angiogenesis, psoriasis, Alzheimer's disease and shock.

CO is administered to inhibit NSAID induced gastrointestinal side-effects. There are various methods known in the art for administering CO. The CO may be administered, for example, as a gas, as a gas dissolved in a liquid or trapped in a carrier, or as a carbon monoxide releasing molecule prodrug (CORM).

CO delivered as a gas is described in WO 03000114 A3, US 2002/0155166 A1, WO 20041043341 A2, US 2004/0052866 A1, WO/096977 A2, WO03/072024 A2, US 2003/0219496 A1, WO 03103585 A2 and US 2005/0048133 A1.

Therapeutic delivery of CO by CORMs is described in WO 2005013691 A1, US 2003/068387A1, WO 2004/0445599, WO03066067A2, US 2004/067261A1, and US Pat 7,011,854.

Therapeutic delivery of CO by heme containing carrier proteins is described in W09422482.

As is known in the art, the NSAID can be covalently conjugated to the CORM. In this instance, the NSAID preferably is attached to the CORM by a linker, which is a bond or is a short (C6-12) organic molecule, preferably an immuluent linker. Suitable bonds which will digest in the stomach include ester and amide bonds. The present invention thus contemplates covalent conjugates of the NSAIDs and CORMs described above, conjugated together by for example an ester bond.

The Particular examples of CORMs include compounds from one of the following classes:
Class 1 - CO containing organometallic complex. Such a compound can be dissolved in physiologically compatible support.
Class 2 - CO containing organometallic complex linked to at least another pharmacologically important molecule. For example, said pharmacologically important molecule is a carrier, a drug (e.g., an anti-inflammatory agent). Furthermore, the CO containing organometallic complex and the at least other pharmacologically important molecule are optionally linked by means of an appropriate spacer.
Class 3 - Supramolecule aggregates made of CO containing organometallic complexes optionally encapsulated e.g. in a cyclodextrin host and/or another appropriate inorganic or organic support.
Class 4 - CO containing inorganic complex bearing ligands, e.g., polidentate ligands, containing N and/or S donors that function as reversible CO carriers.
Class 5 - CO containing inorganic complex bearing ligands, e.g. polidentate ligands, containing N and/or S donors that function as reversible CO carriers, linked to at least another pharmacologically important molecule. For example, the pharmacologically important molecule is a carrier, a drug, (e.g. an anti-inflammatory agent). Furthermore, the CO containing organometallic complex and the at least other pharmacologically important molecule are optionally linked by means of an appropriate spacer.
Class 6 - Organic compounds that release CO either by an enzymatic process or by decarbonylation. Such a compound can be dissolved in physiologically compatible supports.
Class 7 - Organic compounds that release CO either by an enzymatic process or by decarbonylation, e.g., dichloromethane optionally encapsulated either in cyclodextrin hosts and/or other appropriate inorganic or organic supports.

### Class 1- CO containing organometallic complexes dissolved in physiologically compatible supports

This class of compounds comprises either simple 18 electron organometallic carbonyl complexes or modifications thereof designed to improve either their solubility in physiological media or their compatibility with membranes and biomolecules or tissues. The metals that may be used include first transition row biologically active metals (V, Cr, Mn, Fe, Co, Ni, Cu) as well as second (Mo, Ru, Rh, Pd) and third row elements (W, Re, Pt, Au), that appropriately bind the CO ligand. A large number of these compounds bears the cyclopentadienyl ligand (Cp) or derivatives thereof (indenyl, CpR5, and the like) hereby abbreviated as CpR(X), which enable the above-mentioned modifications, and impart some steric protection to the metal center with the corresponding higher reactivity control. The oxidation state of the metal in most of the complexes resembles the one usually found under biological conditions thereby facilitating later metabolization, after CO release.

In the examples listed immediately below, the term "pseudo-halide" is a general name given to mono-anionic ligands isoelectronic with the halides, e.g., thiocyanates, cyanates, cyanides, azides, etc. The term "hydrocarbyl chain" is the general name of a hydrocarbon radical comprising aliphatic CH₂ and/or aromatic residues, e.g., (CH₂)ₙ, n = 2, 3, etc. or (CH₂)ₙ, (C₆H₄)ₘ, C₆H₅CH₂, etc. Alkyl is the general name given to the radical of an aliphatic hydrocarbon chain, e.g. methyl, ethyl, etc. Aryl is the general name given to a radical of an aromatic ring, e.g., phenyl, tolyl, xylyl, etc. Specific examples are depicted in U.S. Patent No. 7,011,854. Examples include:

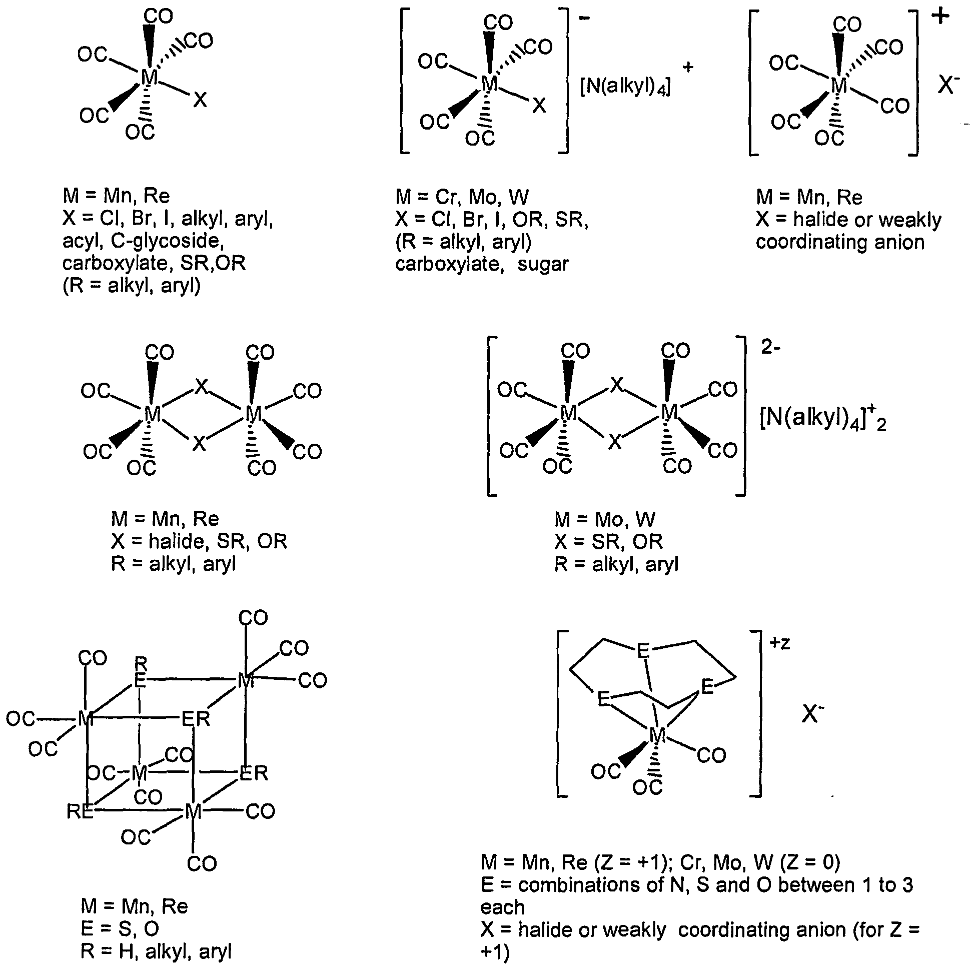

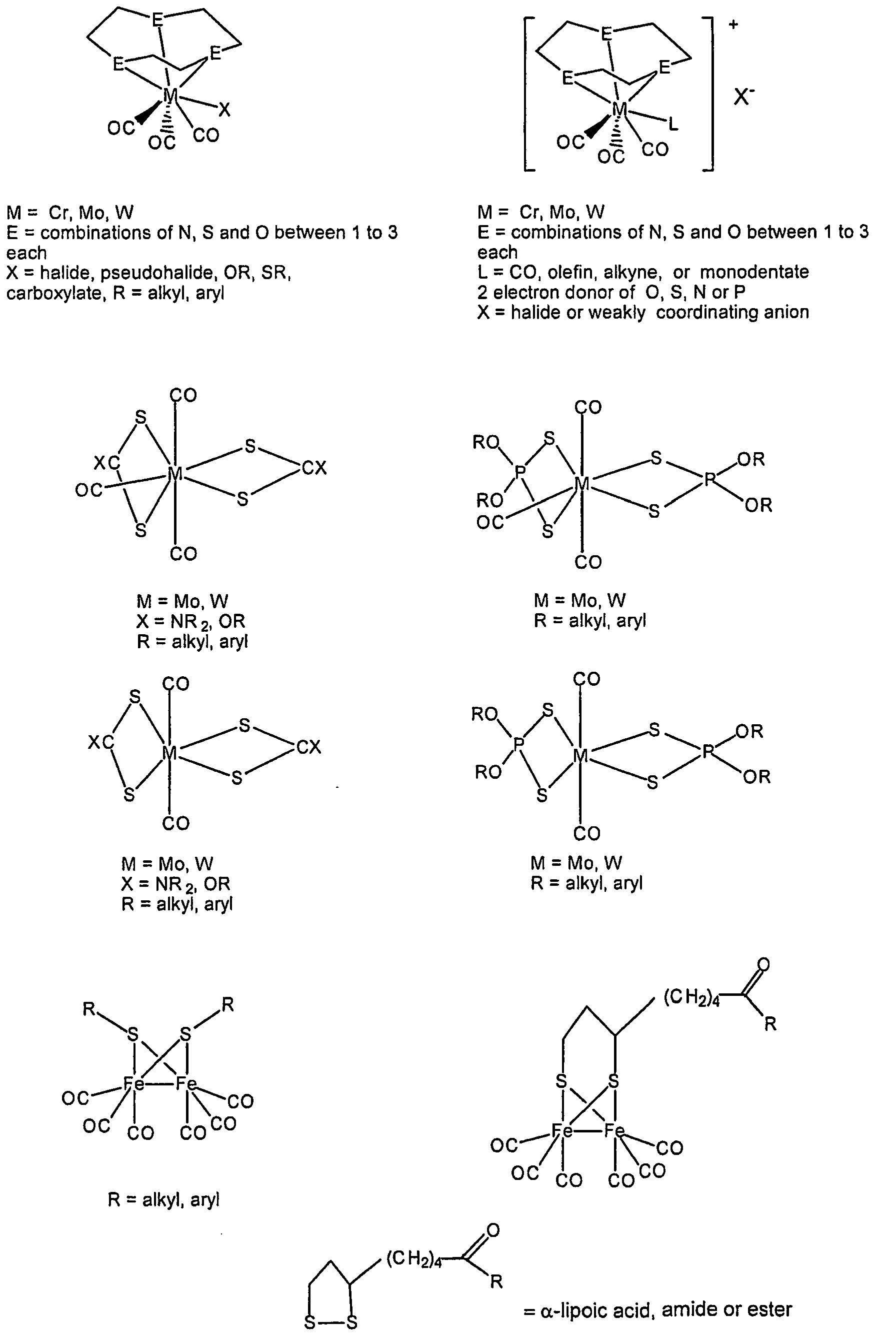

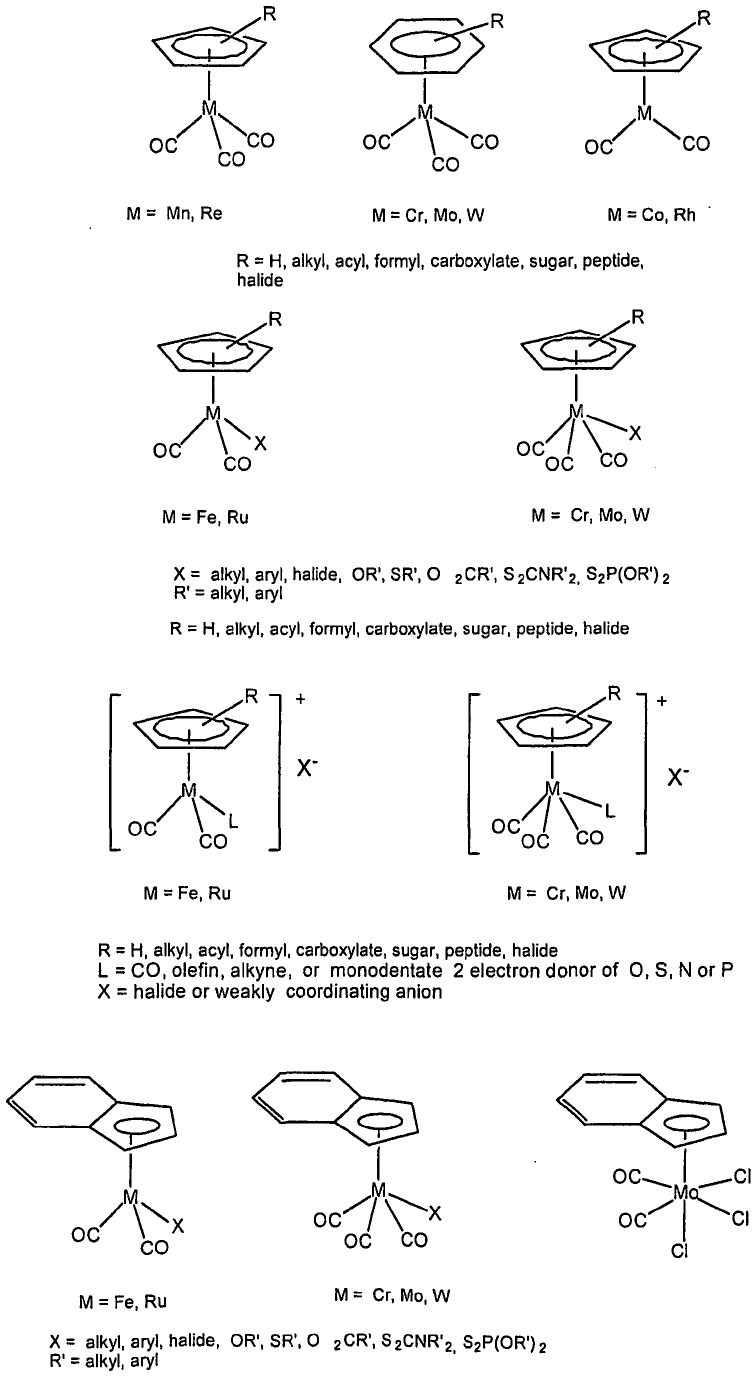

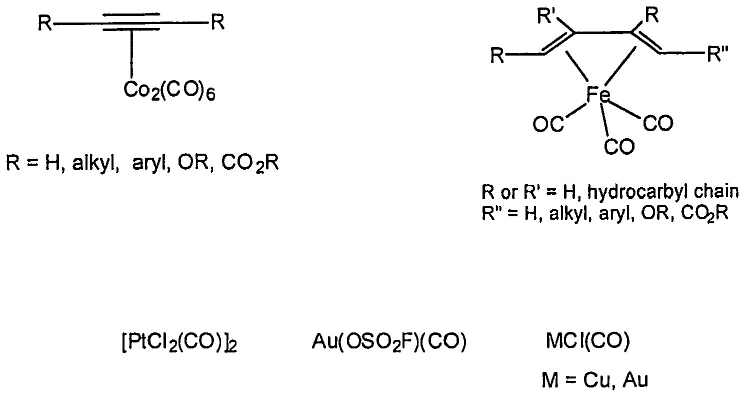

Several modifications can be envisaged to improve higher biological compatibility and solubility. One preferred possibility is to attach carboxylic, peptide or sugar derivatives to the cyclopentadienyl moiety. Examples are depicted for one Mn complex; similar derivatives can be made with compounds containing other metals, as well as for indenyl and other CpR(X) derivatives.

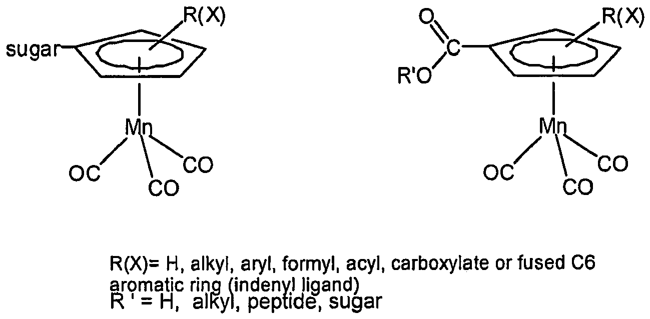

Further embodiments of Class 1 compounds, include:

[Mo(CO)₅Y]Q

wherein Y is bromide, chloride or iodide; and
Q is [NR¹⁻4]⁺
where R¹, R², R³, and R⁴ are each independently alkyl.

As used herein, the term "alkyl," means a C₁-C₁₂ saturated hydrocarbon chain, such as methyl, ethyl, n-propyl, isopropyl, n-butyl, isobutyl, t-butyl, n-pentyl, n-hexyl, n-heptyl, n-octyl, n-nonyl, n-decyl, n-undecyl, or n-dodecyl. In one embodiment, alkyl is a C₁-C₆ or a C₁-C₄ saturated hydrocarbon chain. Specific examples of the foregoing compounds are described in detail WO 2007/073226

Other embodiments of Class 1 compounds include:

[Mo(CO)₅Y]Q

wherein Y is bromide, chloride or iodide; and

Q is [NR₄]⁺, free or complexed with one cyclic polyether molecule or one or more acyclic polyether molecules, or

Na⁺, K⁺, Mg²⁺, Ca²⁺ or Zn²⁺, where each is free or complexed with one cyclic
polyether molecule or one or more acyclic polyether molecules,
wherein
each R is independently H or alkyl.

The cyclic polyether molecule includes, without limitation, crown ethers. In some embodiments, the cyclic polyether includes crown ethers from the 18-crown-6 family or the 15-crown-5 family. The one or more acyclic polyethers are of the polyethylene glycol type and of the formula R¹O(CH₂CH₂O)ₙR² where R¹ and R² are each independently H or alkyl and n is greater than or equal to 1. The acyclic polyether molecules are within the range of pharmaceutically acceptable polyethylene glycols or mono- or dialkyl polyethylene glycols.

When Q is free, Q is not associated with any molecular structure other than a molybdenum complex or molybdenum complexes by electrostatic (ionic) forces. When Q is complexed with one cyclic polyether molecule, or one or more acyclic polyether molecules, these complexed cationic entities are associated with one or more molybdenum anionic complexes by electrostatic bonding. When Q is complexed with acyclic polyethers, an ionic structure results from the interaction between the molybdenum complex or molybdenum complexes and the complexes formed between the acyclic polyethers and the NR₄⁺ or metal cation. The NR₄⁺ or metal cation may accommodate a variable, yet definite and controllable, number of non-covalently bound acyclic polyether molecules giving rise to different polymorphs or solvates. In one embodiment, the NR₄⁺ or metal cation non-covalently binds up to twelve acyclic polyether molecules at one time.

As used herein, the term "alkyl," means a C₁-C₁₂ saturated hydrocarbon chain, such as methyl, ethyl, n-propyl, isopropyl, n-butyl, isobutyl, t-butyl, n-pentyl, n-hexyl, n-heptyl, n-octyl, n-nonyl, n-decyl, n-undecyl, or n-dodecyl. In one embodiment, alkyl is a C₁-C₈ or a C₁-C₆ or a C₁-C₄ saturated hydrocarbon chain.

In some embodiments, Q is complexed with one cyclic polyether molecule or one or more acyclic polyether molecules. In some embodiments, the one cyclic polyether molecule includes crown ethers from the 18-crown-6 family or the 15-crown-5 family. In other embodiments Q is complexed by one or more acyclic polyethers in a coordination sphere comprising from four to twelve oxygen atoms of the ethyleneglycol or polyethylene glycol type chains. In yet another embodiment, Q is complexed by six, eight, or twelve acyclic polyether molecules. In another embodiment, Q is complexed by three acyclic diethers. In further embodiments, Q is complexed with one, two, or three polyether molecules.

In some embodiments, Q is complexed with more than one acyclic polyether molecules of the formula R¹O(CH₂CH₂O)ₙR² where R¹ and R² are each independently H or alkyl, n is greater than or equal to 1, and the polyether molecules are within the range of pharmaceutically acceptable polyethylene glycols or mono- or dialkyl polyethylene glycols. In further embodiments, when Q is complexed with more than one ether of the formula R¹O(CH₂CH₂O)ₙR², each R¹ and R² of each polyether molecule is independently H or alkyl, so that each polyether of the formula R¹O(CH₂CH₂O)ₙR² may be different and each R¹ or R² may be different than an R¹ or R² in another polyether molecule.

In further embodiments, specific acyclic ethers include, without limitation, monoglyme, diglyme, triglyme, PEG 400, PEG 1000, PEG 2000, PEG 3000 and PEG 4000, and methylPEG400.

Specific examples of the foregoing compounds are described in WO 2007/073225. Examples include:

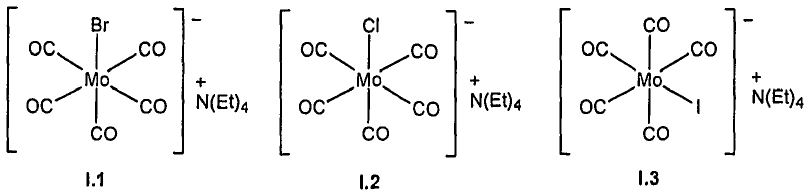

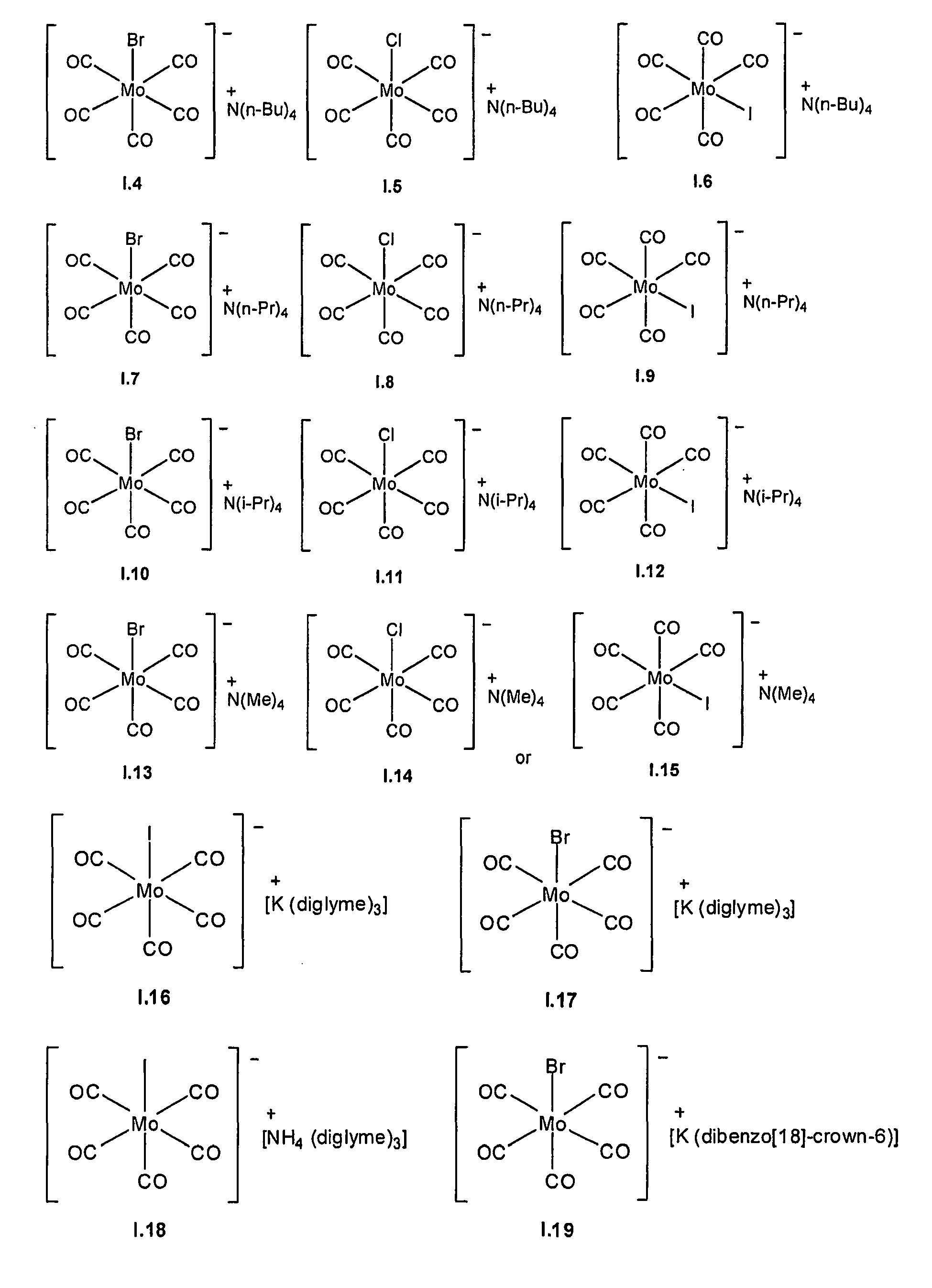

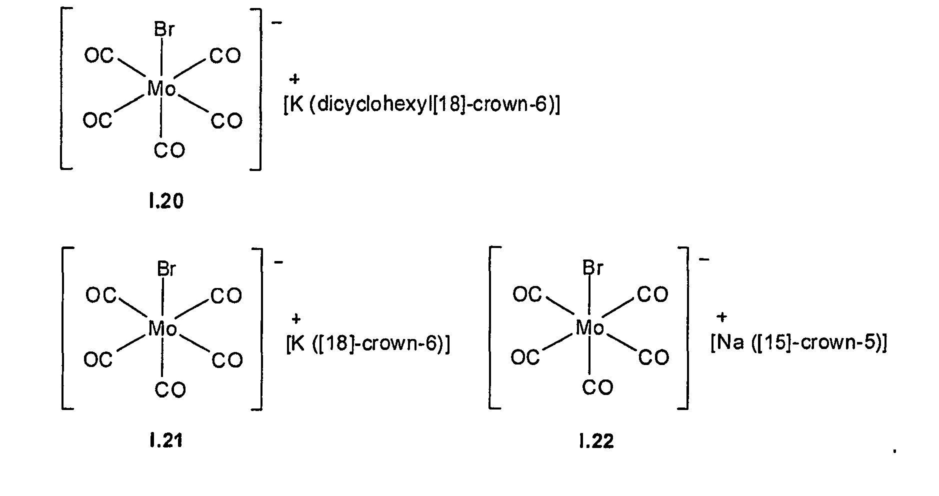

### Class 2- CO containing organometallic complexes linked to other pharmacologically important molecules.

This class of compounds takes advantage of the synergistic effects arising from the combination of two biologically active molecules, which both have beneficial effects. Examples for such drug-drug conjugates have been described in U.S. Patent 6,051, 576.

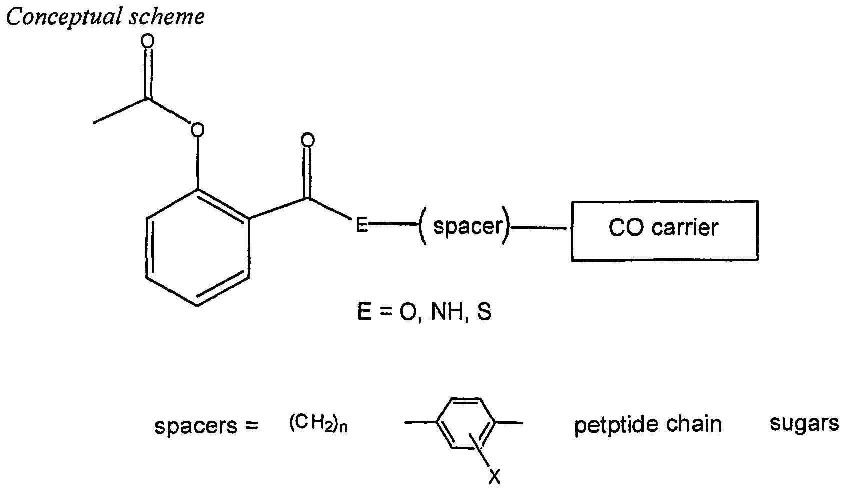

The above mentioned spacers comprise a variety of functions under the following specifications: the value of "n" in the linear hydrocarbon chain is an integer more specifically 1, 2, 3, 4: X is a general symbol for a substituent at the aromatic ring, namely, alkyl, aryl, alkoxy, aryloxl, halogen atom, thiolate; "peptide chain" represents a short chain of natural amino acids ranging from 1 to 4; by "sugars" it is meant the use of a mono-, di- or polysaccharide either protected or modified with adequate protection to increase lipophilicity and/or assure chemical stability of the drug-drug conjugate molecule, for example, with protective groups, such as esters, acetals, and silyl derivatives.

The definition of X given immediately above can be extended to carboxylates and amino acids in the cases where X is directly bound to the metal as in some of the leading examples depicted in the next scheme.

### Leading examples:

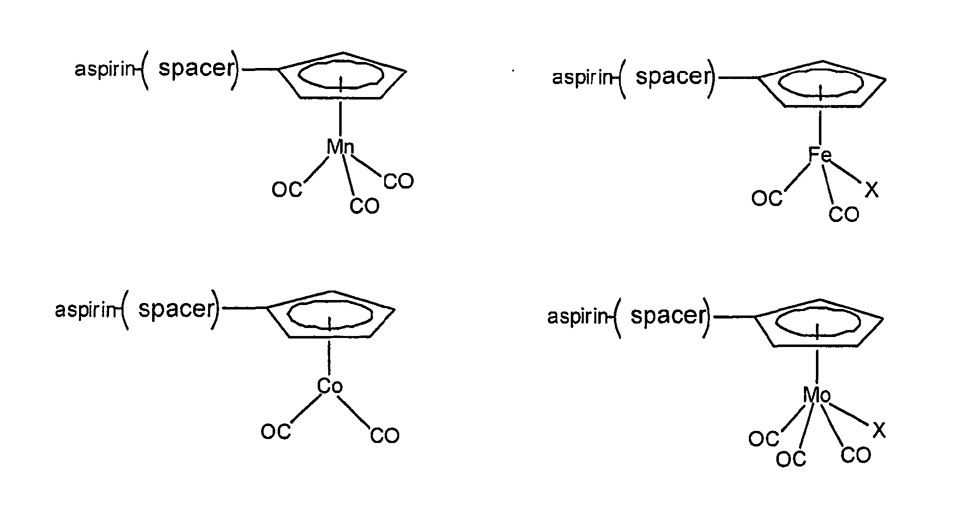

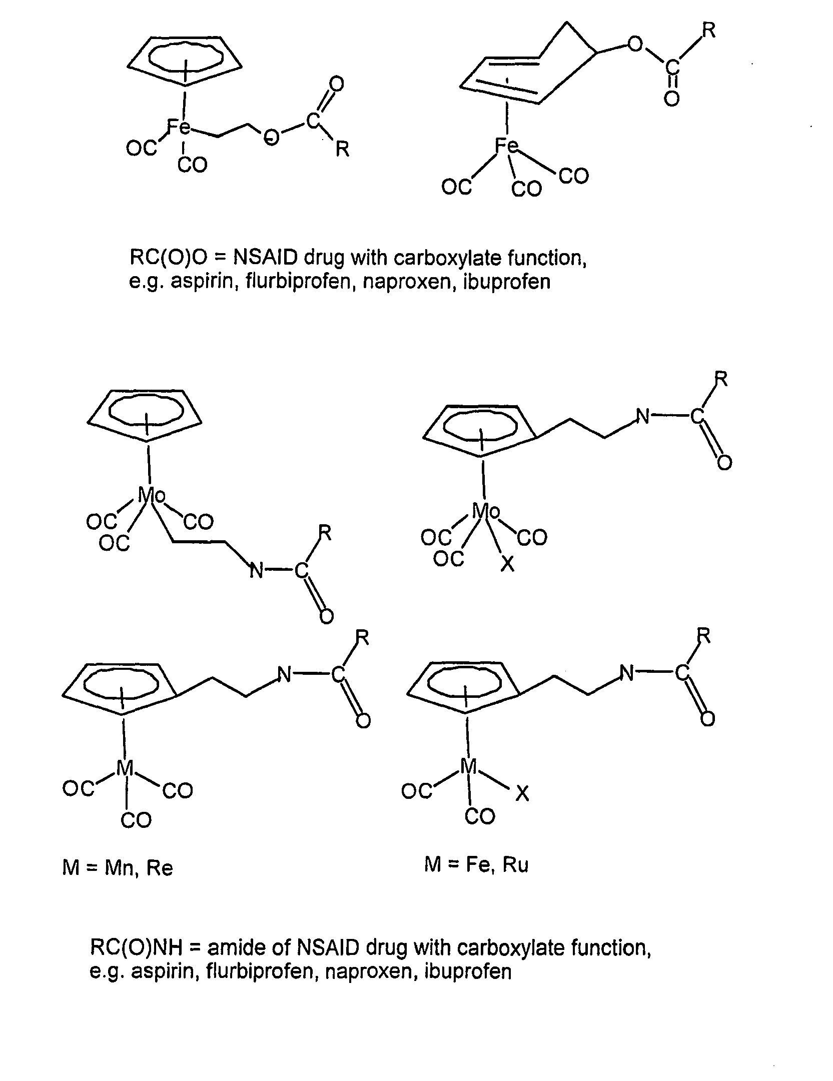

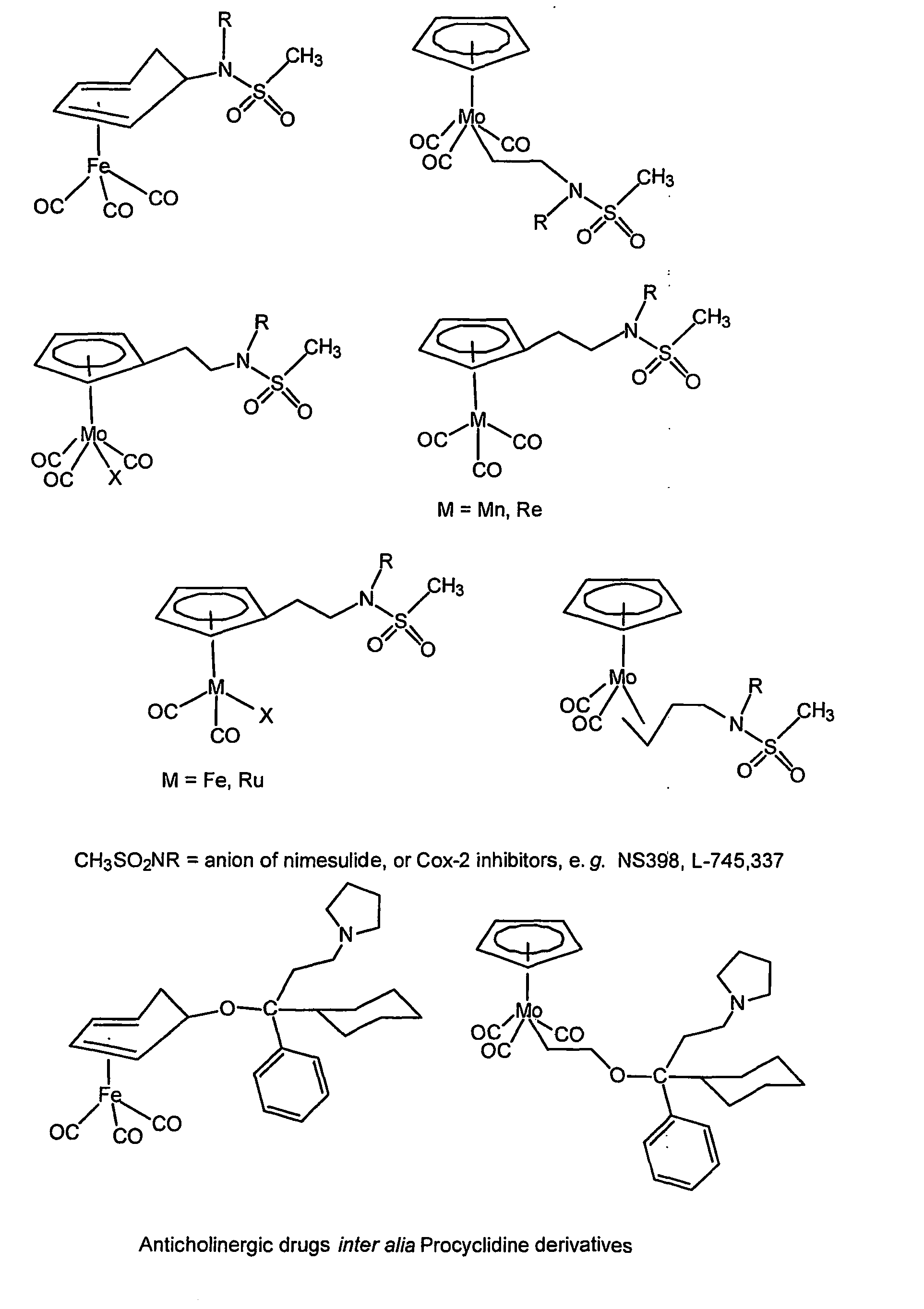

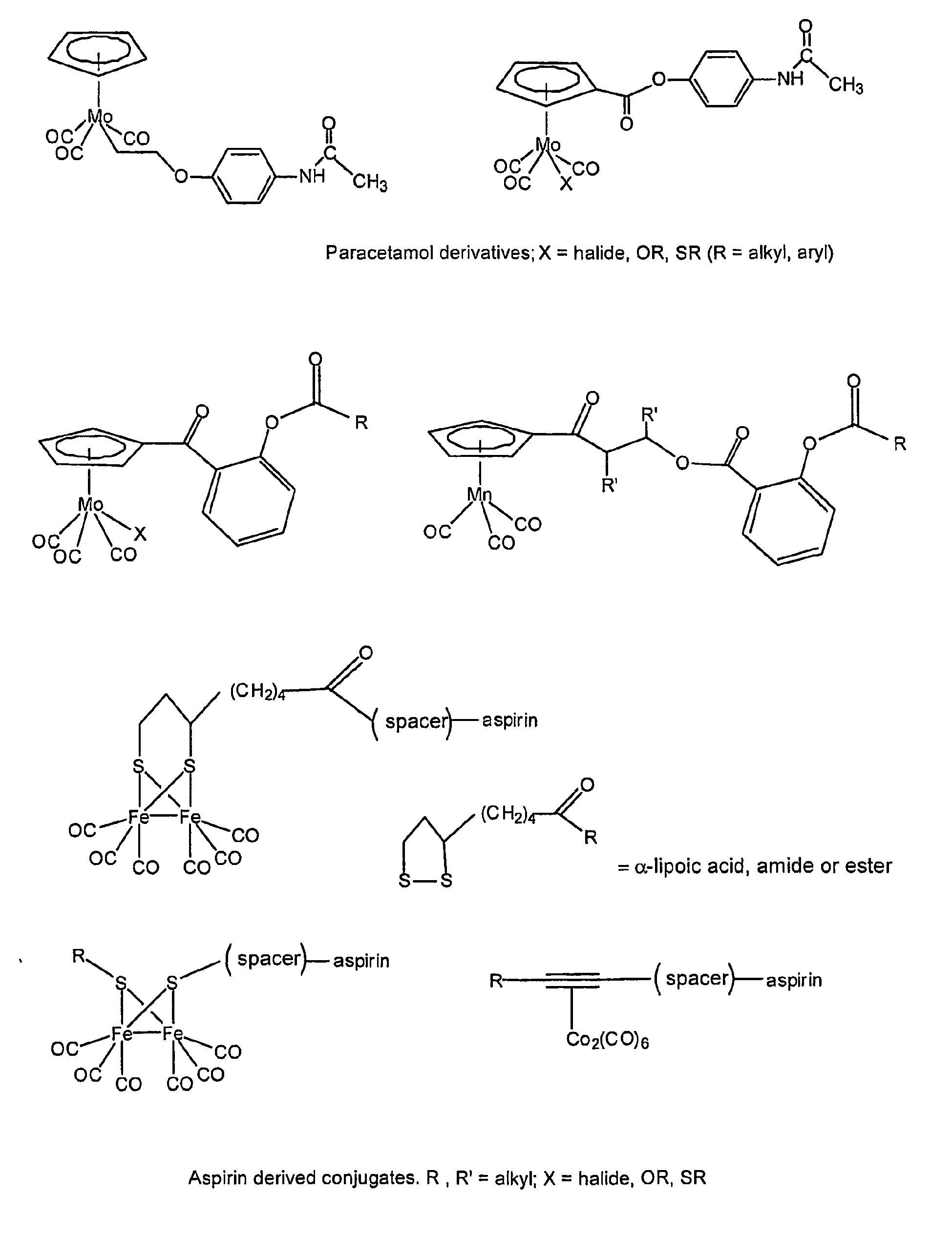

A second group of compounds bears the bioactive molecule, e.g. aspirin, diphosphonate, bound directly to the metal, which can be achieved in several different manners as schematized below for the case of some iron and molybdenum cyclopentadienyl carbonyls, among others. The term "hydrocarbyl chain" is the general name of a hydrocarbon radical comprising aliphatic CH₂ and/or aromatic residues, e.g., (CH₂)ₙ, n = 2, 3, etc. or (CH₂)ₙ, (C₆H₄)ₘ, C₆H₅CH₂,

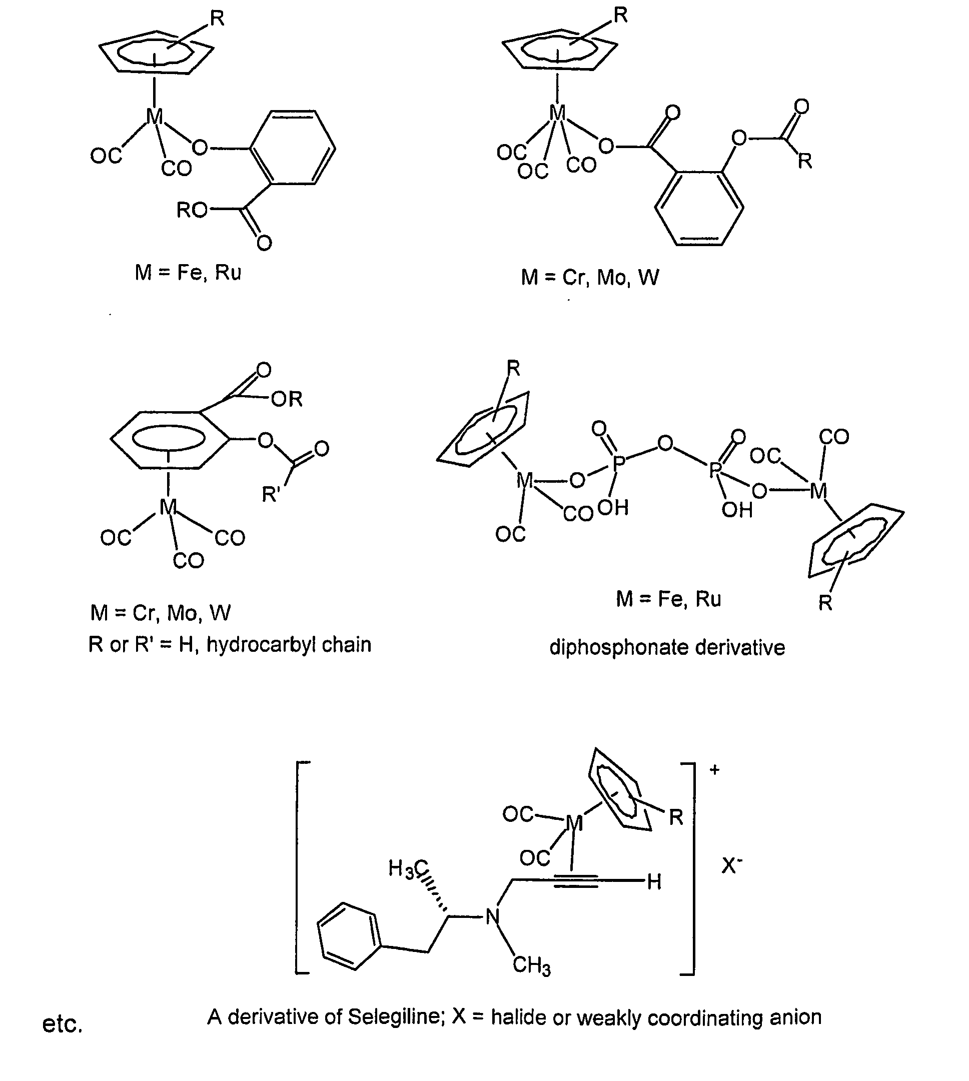

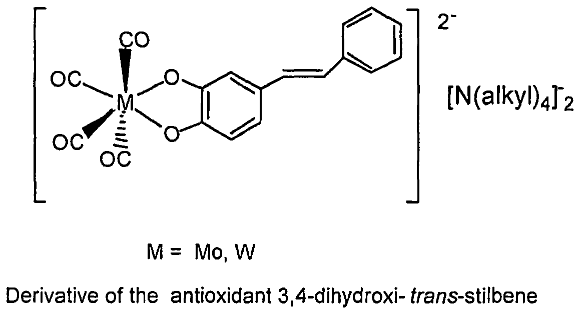

### Class 3: Encapsulated supramolecular aggregates made of CO containing organometallic complexes.

Controlled delivery of drugs into the organism is an important issue, especially in the case of drugs, which have undesired toxic effects if present systemically or at high local concentrations. CO release is a potential problem inasmuch as it can be toxic at high concentrations (see above). For certain applications, a slow release of CO in the blood or in specific target tissues is desirable. Encapsulation within host molecules that are non-toxic is one way to achieve a sustained release of active drugs in the organism. This strategy minimizes the undesired effects that may result from abrupt increases in the concentration and/or availability of a potentially toxic drug.

Cyclodextrins are well known hosts for many drugs and organic molecules and, recently have been applied to host organometallic molecules and enhance their delivery through physiological barriers or membranes. In this respect cyclodextrin has been found to be beneficial for increasing delivery of lipophilic drugs at the skin barrier. [T. Loftsson, M. Masson, Int. J. Pharm. 2001, 225, 15]. Cyclodextrin mediated supramolecular arrangements protect organometallic molecules for prolonged time periods and mask their reactivity, thereby increasing their selectivity towards specific agents. Octahedral, mononuclear carbonyl complexes exemplified under Class 1 above, such as for example, [M(CO)₆]^{0,+} or [M(CO)₅X]^{0,+} are small and fit in the cavity of β-or γ-cyclodextrin.

The hydrophobic ring of other carbonyl complexes exemplified under Class 1 above, fits inside ß- or γ-cyclodextrin, or similar structures, with the CO groups facing the reaction medium and the organic ligands buried in the cavity. The resulting reduction in reactivity allows for the extension of the range of therapeutic CO-releasing complexes to cationic and anionic ones. Such charged complexes are more reactive and lose CO faster than the neutral ones when unprotected. In some embodimend

Liposomes and other polymeric nanoparticle aggregates are also useful carriers to target the delivery of CO-releasing organometallic complexes and the combined use of cyclodextrins with such aggregates has been considered as a very promising possibility for drug release. [D. Duchêne, G. Ponchel, D. Wouessidjewe, Adv. Drug Delivery Rev. 1999, 36, 29.]

### Conceptual examples

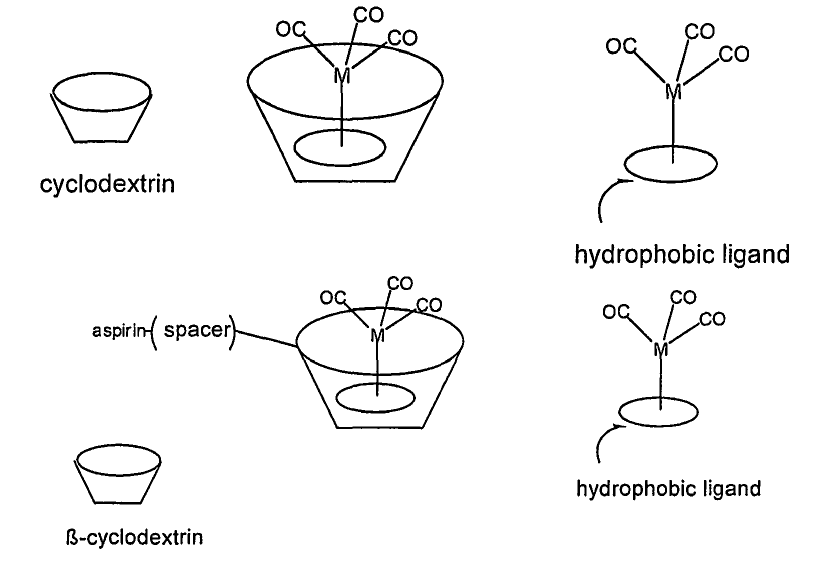

Some of the examples cover organometallic molecules as (C₆H₆-ₓRₓ)M(CO)₃ (M = Cr, Mo, W); (CpR₅)M(CO)₃X (M = Cr, Mo, W); (CpR₅)M(CO)₂X (M = Fe, Ru); (CpR₅)M(CO)₂ (M = Co, Rh) where R represents H, alkyl or other small functional group like methoxide, halide, carboxylic esters.

Other examples include encapsulates of Mn(CO)₅Br and [Mo(CO)₅Br]NEt₄ in modified cyclodextrin, 2,3,6-tri-*O*-methyl-β-cyclodextrin (also known as TRIMEB). These encapsulates are prepared by methods well established in the art. For example, solutions of Mn(CO)₅Br or [Mo(CO)₅Br]NEt₄ in dichloromethane are added to solutions of a commercial heptakis-2,3,6-tri-*O*- methyl-β-cyclodextrin (trimeb) with stirring for approximately 3 hours. The products are obtained after evaporation of the solvent to dryness. The stoichiometry of inclusion is 1:1 and the compounds retain water of crystallization which can be quantified by chemical analysis (Reference for Mn(CO)₅ Br:Herrmann-Brauer (1997), Bromo(pentacarbonyl)manganese, Synthetic Methods of Organometallic and Inorganic Chemistry (ed. Herrmann, W. A. Stuttgart); Reference for [Mo(CO)₅Br]NEt₄: Burgmayer, S. J. N. & L., T. J. (1985), Tetraethylammonium molybdenum pentacarbonyl bromide. Inorganic Chemistry 24, 2224-2230),

Mesoporous materials are chemically inert three dimensional molecules with infinite arrays of atoms creating channels and cavities of well defined pore size. These molecules are well suited to host organic and organometallic molecules in their pores. In the presence of biological fluids, smaller molecules undergoing acid-base and/or polar interactions with the inner walls of the pores slowly displace the included drugs, resulting in a controlled delivery of the active principle. Such aggregates have been prepared from M41 S materials using organometallic molecules like those depicted under system 1 above. Examples include MCM-41 (linear tubes) and MCM-48 (cavities and pores).

### Class 4- CO containing inorganic complexes bearing ligands containing N and/or S donors that function as reversible CO carriers.

Classical inorganic complexes bearing macrocyclic ligands on an equatorial plane of an octahedral coordination sphere are known to reversibly bind CO much in the same way as hemoglobin. The capacity to bind CO can be "tuned" by the nature of both the macrocycle and the ancilliary ligand trans to CO. A similar behavior has also been reported for other Fe(II) complexes bearing ligands that are much simpler than the porphyrin macrocycles that are the CO acceptor sites in hemoglobin and other heme containing proteins. In order to develop suitable CO delivering drugs, the later type of non-hemic complexes was chosen to avoid interference with the biological heme carriers, heme metabolism, and potential toxicity of heme or heme-like molecule. The complexes selected bear bidentate N donors (diamines, diglyoximes) or bidentate N,S donors of biological significance, like aminothiols or cysteine. Ancilliary ligands are N donors also of biological significance like imidazole, hystidine, and others. The complexes are soluble in aqueous media.

In the examples immediately below, the term pyridines refers to derivatives of the C₅H₅N ring (pyridine) bearing alkyl (R), alkoxy (OR), carboxy (C(O)OR), nitro (NO₂), halogen (X), substituents directly bound to the one or more positions of the C5 carbon ring, e.g. CH₃C₅H₄N, O₂NC₅H₄N. Amino-thiols refers to compounds bearing both the NH₂ (amino) and SH (thiol) functions bound to a hydrocarbon skeleton, e.g. H₂NCH₂CH₂SH, 1,2-C₆H₄(NH₂)(OH). A similar definition applies to amino alcohols, whereby the SH function is replaced by the OH (alcohol) function. The term amino acids refers to naturally occurring single amino acids coordinated in a bidentate fashion by the NH₂ and the COO functions as schematically depicted. Glyoximes are bidentate N donors, bearing either alkyl or aryl substituents on the hydrocarbon chain binding the two N atoms, as depicted in the first example below for a diaryl glyoxime. Diimines present a similar structure whereby the OH groups in the diglyoximes are replaced by alkyl or aryl groups. An extension of this family of ligands includes also 2,2'-bypiridines, e.g., 2,2'-dipyridyl, and phenanthrolines.

### Examples:

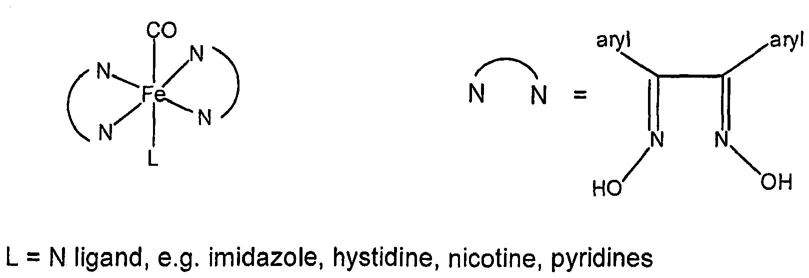

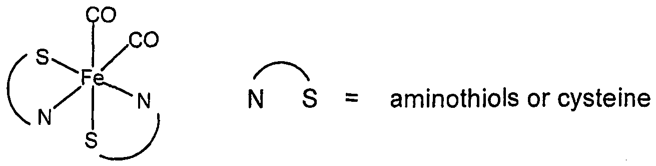

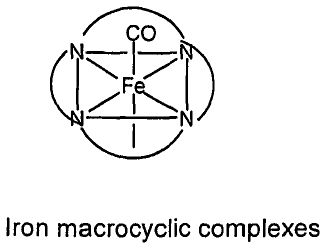

### Class 5- CD containing inorganic complexes bearing ligands containing N and/or S donors that function as reversible CO carriers, modified by linkage to other pharmacologically important molecules.

Following the lines of thought outlined above for Class 2 compounds, new CO carriers of the type described as Class 4, but modified by linking the ligands to other biologically active molecules via an appropriate spacer, were prepared.

### Examples:

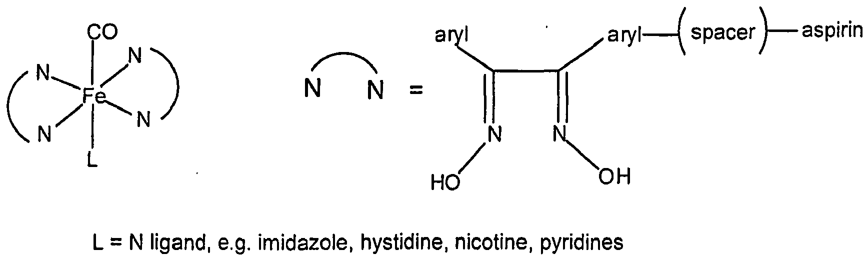

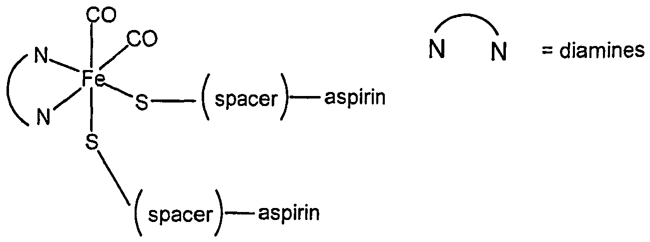

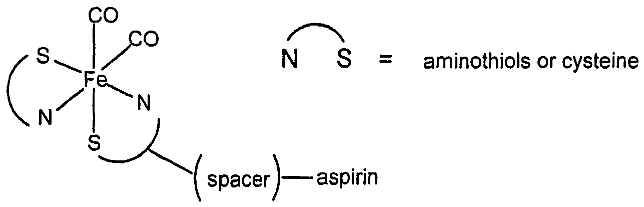

### Class 6- Organic compounds that release CO either by an enzymatic process or by decarbonylation.

In spite of the fact that decarbonylation is not a very common type of reaction in organic chemistry, some organic compounds are known to liberate CO upon treatment with either bases, acids, or radical initiators depending on their nature. These substances fall into the following groups: polyhalomethanes of the general form CHₙXyX' _{4-(n+y)} (X and or X' = F, Cl, Br, I) trichloroacetic acid, and its salts, organic and inorganic esters and sulfinates thereof, triaryl carboxylic acid, formic acid, oxalic acid, α-hydroxyacids and α -ketoacids, esters and salts thereof, under acid conditions; trialkyl and trialkoxybenzaldehydes under acid catalysis; aliphatic aldehydes with radical initiators, e.g., peroxides or light. For the polyhalomethanes, the values of n and y vary in the following way: for n = 0, y = 1,2, 3, 4; for n = 1, y = 1,2,3; for n = 2, y = 1, 2; for n = 3, y = 1. In the above examples, the term "salt" applies to the ionic derivative of the conjugate base of a given protonic acid, namely a carboxylate, with a main group element ion, namely Na⁺, K⁺. Alkyl is the general name given to the radical of an aliphatic hydrocarbon chain, e.g. methyl, ethyl, propyl, butyl, etc. The alkyl group can be branched or straight chain. Aryl is the general name given to a radical of an aromatic ring, e.g., phenyl, tolyl, xylyl, etc. The aryl group will typically have about 6 to about 10 carbon atoms. Ester is the general name given to the functional group W-C(O)OR (where R = alkyl, aryl).

The first two categories produce dichlorocarbene, which, under physiological conditions, will be metabolized to CO. In the case of dichloromethane, cytochrome P-450 has been shown to be responsible for the liberation of CO *in vivo.*

The third group of compounds releases CO under acid catalysis and is sensitive to the aryl substitution pattern. Most likely this is also true for the fourth group which includes trialkyl and triaryl substituted aldehydes. Strong activating groups on the aryl ring favor CO liberation under acid conditions. More importantly, the radical initiated decomposition of aliphatic aldehydes, induced by peroxides or light, produces CO under very mild conditions. The value of "n", the number of substituents (alkyl, aryl, alkoxy, aryloxy) on the aromatic ring, can vary from 0 to 5, preferably 1, 2, or 3.

### Examples:

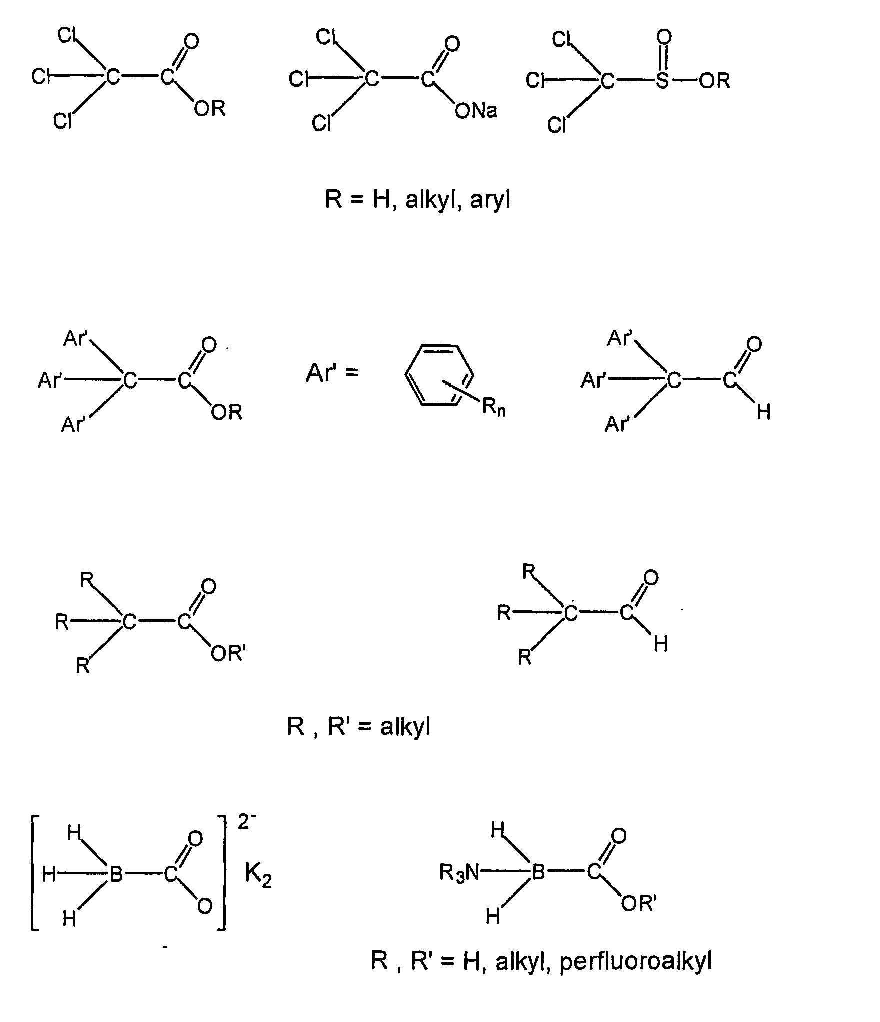

Other examples of CORM aldehydes include: 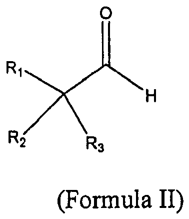 wherein R₁, R₂ and R₃ are each independently selected from H, alkyl, substituted alkyl, cycloalkyl, substituted cycloalkyl, heterocyclyl, substituted heterocyclyl, alkylheterocyclyl, substituted alkylheterocyclyl, alkenyl, substituted alkenyl, aryl, substituted aryl, heteroaryl, substituted heteroaryl, alkylaryl, substituted alkylaryl, hydroxy, alkoxy, amino, alkylamino, mercapto, alkylmercapto, aryloxy, substituted aryloxy, heteroaryloxy, substituted heteroaryloxy, alkoxycarbonyl, acyl, acyloxy, acylamino, alkylsulfonyl, alkylsulfinyl, F, Cl, Br, NO₂ and cyano; or two or more of R₁, R₂ and R₃ are taken together to form a substituted or unsubstituted carbocyclic or heterocyclic ring structure.

"alkyl" refers to straight or branched chain saturated hydrocarbyl groups having up to 20 carbon atoms, and "substituted alkyl" refers to alkyl groups bearing one or more substituents selected from amino, alkylamino, hydroxy, alkoxy, mercapto, alkylmercapto, aryl, aryloxy, alkoxycarbonyl, acyl, acyloxy, acylamino, F, Cl, Br, NO₂ cyano, sulfonyl, sufinyl and similar substituents known to those of skill in the art. "Cycloalkyl" refers to saturated hydrocarbyl groups containing one or more rings and having in the range of 3 to 12 carbon atoms, and "substituted cycloalkyl" refers to cycloalkyl groups further bearing one or more substituents as set forth above. "Heterocyclyl" refers to cyclic groups containing one or more rings including one or more heteroatoms (*e.g*., N, O or S) as part of the ring structure and having in the range of 3 to 12 ring atoms, and "substituted heterocyclyl" refers to heterocyclyl groups further bearing one or more substituents as set forth above. "Alkylheterocyclyl" refers to alkylsubstituted heterocyclyl groups, and "substituted alkylheterocyclyl" refers to alkylheterocyclyl groups further bearing one or more substituents as set forth above. "Alkenyl" refers to straight or branched chain hydrocarbyl groups having at least one carbon-carbon double bond, and having in the range of 2 to 20 carbon atoms, and "substituted alkenyl" refers to alkenyl groups further bearing one or more substituents as set forth above. "Aryl" refers to aromatic groups having in the range of 6 up to about 14 carbon atoms, and "substituted aryl" refers to aryl groups further bearing one or more substituents as set forth above. "Heteroaryl" refers to aromatic groups containing one or more heteroatoms (*e.g*., N, O or S) as part of the ring structure, and having in the range of 5 up to about 13 carbon atoms, and "substituted heteroaryl" refers to heteroaryl groups further bearing one or more substituents as set forth above. "Alkylaryl" refers to alkylsubstituted aryl groups, and "substituted alkylaryl" refers to alkylaryl groups further bearing one or more substituents as set forth above.

"Hydroxy" refers to the group OH. "Alkoxy" refers to a group -OR, wherein R is an alkyl group as defined above. "Amino" refers to the group NH₂. "Alkylamino" refers to a group NHR or -NRR', where R and R' are independently chosen from alkyl or cycloalkyl groups as defined above. "Mercapto" refers to the group SH. "Alkylmercapto" refers to the group S-R, where R represents an alkyl or cycloalkyl group as defined above. "Aryloxy" refers to a group -OAr, wherein Ar is an aryl group as defined above, and "substituted aryloxy" refers to aryloxy groups further bearing one or more substituents as set forth above. "Heteroaryloxy" refers to a group -OHt, wherein Ht is a heteroaryl group as defined above, and "substituted heteroaryloxy" refers to heteroaryloxy groups further bearing one or more substituents as set forth above. "Alkoxycarbonyl" refers to a group -C(O)-OR, wherein R is an alkyl group as defined above.

"Acyl" refers to a group -C(O)-R, where R is H, alkyl, substituted alkyl, cycloalkyl, substituted cycloalkyl, alkenyl, substituted alkenyl, aryl, substituted aryl, heteroaryl or substituted heteroaryl, as defined above. "Acyloxy" refers to a group -O-C(O)-R, where R is H, alkyl, substituted alkyl, cycloalkyl, substituted cycloalkyl, alkenyl, substituted alkenyl, aryl, substituted aryl, heteroaryl or substituted heteroaryl, as defined above. "Acylamino" refers to a group -NR'C(O)R, where R and R' are each independently chosen from H, alkyl, substituted alkyl, cycloalkyl, substituted cycloalkyl, alkenyl, substituted alkenyl, aryl, substituted aryl, heteroaryl or substituted heteroaryl, as defined above. "Alkylsulfonyl" refers to a group -S(O)₂R, where R represents an alkyl or cycloalkyl group as defined above. "Alkylsulfinyl" refers to a group -S(O)R, where R represents an alkyl or cycloalkyl group as defined above.

Non-limiting examples of CORM aldehydes include the following: trimethylacetaldehyde (compound 1) 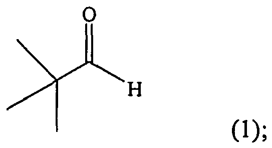 2,2-dimethyl-4-pentenal (compound 2) 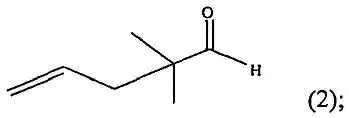 4-ethyl-4-formyl-hexanenitrile (compound 3) 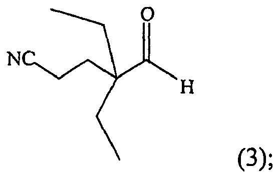 3-hydroxy-2,2-dimethylpropanal (compound 4) 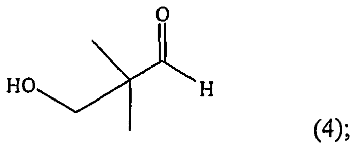 2-formyl-2-methyl-propylmethanoate (compound 5) 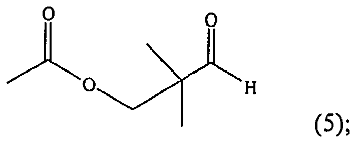 2,2-dimethyl-3-(p-methylphenyl)propanal (compound 6) 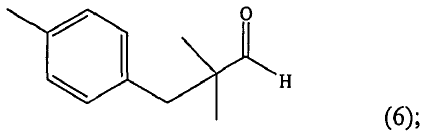 2-methyl-2-phenylpropionaldehyde (compound 7) 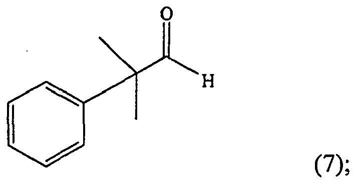 and 2-ethyl-2-methyl-propionaldehyde (compound 8)

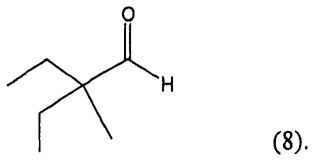

The most common reactions known for the decarbonylation of aldehydes require drastic conditions, such as strong acidic or basic conditions, high temperatures together with ultraviolet light, radical initiators and/or the presence of a metal catalyst (Jerry March, Advanced Organic Chemistry, Reactions, Mechanisms and Structure, John Wiley & Sons, 4th Ed., 1992). However, highly branched aldehydes have been observed to decarbonylate at room temperature when irradiated by ultraviolet light (Berman et al., J. Am. Chem. Soc., 85:4010-4013 (1963); Conant et al., J. Am. Chem. Soc. 51:1246-1255 (1929)). The loss of carbon monoxide from tertiary aldehydes leads to tertiary radicals, which are more stable than primary or secondary radicals due to resonance stabilization by hyperconjugation. Hyperconjugation includes the stabilization that results from the interaction of electrons in a σ-bond (usually C-H or C-C) with an adjacent empty (or partially filled) p-orbital or π-orbital to give an extended molecular orbital that increases the stability of the system. Thus, decarbonylation is favored in tertiary aldehydes, as compared to primary and secondary aldehydes.

While not to be bound by any particular theory, the following equation 1 shows a proposed mechanism for the decarbonylation of tertiary aldehydes (exemplified by trimethylacetaldehyde (compound 1)) by reactive oxygen species, generating carbon monoxide and a stabilized tertiary radical:

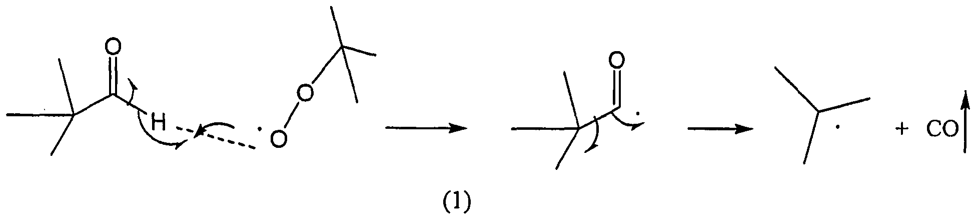

Accordingly, in certain embodiments, the aldehyde is a tertiary aldehyde. In such embodiments, the aldehyde is a compound of the above Formula II in which R₁, R₂ and R₃ are each independently selected from alkyl, substituted alkyl, cycloalkyl, substituted cycloalkyl, heterocyclyl, substituted heterocyclyl, alkylheterocyclyl, substituted alkylheterocyclyl, alkenyl, substituted alkenyl, aryl, substituted aryl, heteroaryl, substituted heteroaryl, alkylaryl, substituted alkylaryl, hydroxy, alkoxy, amino, alkylamino, mercapto, alkylmercapto, aryloxy, substituted aryloxy, heteroaryloxy, substituted heteroaryloxy, alkoxycarbonyl, acyl, acyloxy, acylamino, alkylsulfonyl, alkylsulfinyl, F, Cl, Br, NO₂ and cyano; or two or more of R₁, R₂ and R₃ are taken together to form a substituted or unsubstituted carbocyclic or heterocyclic ring structure.

In some instances, for example, to improve the *in vivo* stability, bioavailability, or pharmacokinetic properties of a therapeutic aldehyde, the aldehyde is administered in the form of a derivative, or a protected form thereof. The derivative serves as a source of the free or unmodified aldehyde *in vivo* and/or releases CO *in vivo* itself. In certain embodiments, an aldehyde derivative is generated that acts as a prodrug, a pharmacologically inactive chemical entity that, when chemically transformed or metabolised in an animal, is converted into a pharmacologically active substance. The generation of the therapeutically effective molecule (*i.e*., the aldehyde) from the prodrug occurs prior to, during or after reaching the site of action within the body (Bundgaard et al., Int. J. Pharm. 13:89-98 (1983)). Release of the aldehyde from the prodrug generally occurs via chemical or enzymatic lability, or both, within the body system.

Examples of aldehyde prodrugs that are chemically labile include, without limitation, non-cyclic chain compounds that exist in equilibrium in physiological media, such as Mannich base derivatives, imines, oximes, amidines, hydrazones and semicarbazones (WO 2006/012215; Herrmann et al., Chem. Commun. 2965-2967 (2006); Deaton et al., Bioorg. Med. Chem. Lett. 16:978-983 (2006)), and ring chain tautomeric prodrugs such as 1,3-X,N-heterocycles (X = O, S, NR) (Valters et al., Adv. Heterocycl. Chem 64:251-321 (1995); Valters et al., Adv. Heterocycl. Chem. 66:1-71 (1996)) that are prepared from the reaction of difunctional compounds with aldehydes. From the ring chain equilibria of these derivatives, the open form undergoes hydrolysis to give the bioactive molecule. In both cases, the ratios of the species involved in the equilibria of these systems are strongly influenced by the steric and electronic characters of the substituents.

An alternative strategy is to generate prodrugs that are converted to the pharmacologically active compound by an enzymatic process (Bernard Testa & Joachim M. Mayer, Hydrolysis in Drug and Prodrug Metabolism, Chemistry, Biochemistry and Enzymology WILEY-VCH, 2003). There are several types of chemical groups such as, for example, esters, amides, sulphates and phosphates, that are readily cleaved by esterases, aminases, sulphatases and phosphatases, respectively. Pharmacologically active aldehydes are released by the action of esterases and amidases on a variety of compounds that include acyloxyalkyl esters, *N*-acyloxyalkyl derivatives, *N*-Mannich bases derivative, *N*-hydroxymethyl derivatives, and others. In some instances, to facilitate hydrolysis when the prodrug is a poor substrate for the aldehyde-generating enzyme, the carrier is modified with electron withdrawing or donating groups.

As recognized by those skilled in the art, organic aldehydes undergo a variety of reactions that render the aldehyde chemically protected. By way of non-limiting example, in various embodiments, organic aldehydes are protected by conversion to the corresponding acetal, hemiacetal, aminocarbinol, aminal, imine, enaminone, imidate, amidine, iminium salt, sodium bissulfite adduct, hemimercaptal, dithioacetal, 1,3-dioxepane, 1,3-dioxane, 1,3-dioxalane, 1,3-dioxetane, α-hydroxy-1,3-dioxepane, α-hydroxy-1,3-dioxane, α-hydroxy-1,3-dioxalane, α-keto-1,3-dioxepane, α-keto-1,3-dioxane, α-keto-1,3-dioxalane, α-keto-1,3-dioxetane, macrocyclic ester/imine, macrocyclic ester/hemiacetal, oxazolidine, tetrahydro-1,3-oxazine, oxazolidinone, tetrahydro-oxazinone, 1,3,4-oxadiazine, thiazolidine, tetrahydro-1,3-thiazine, thiazolidinone, tetrahydro-1,3-thiazinone, imidazolidine, hexahydro-1,3-pyrimidine, imidazolidinone, tetrahydro-1,3-pyrimidinone, oxime, hydrazone, carbazone, thiocarbazone, semicarbazone, semithiocarbazone, acyloxyalkyl ester derivative, O-acyloxyalkyl derivative, N-acyloxyalkyl derivative, N-Mannich base derivative, or N-hydroxymethyl derivative. The exemplary schemes in equations 2-12 below also illustrate how many such prodrugs release the active aldehyde *in vivo* (*e.g.,* via hydrolytic or enzymatic hydrolysis).

In certain embodiments, the protected organic aldehyde is an imine. Those skilled in the art recognize that such derivatives are obtained in a variety of ways, such as, for example, by the methods described by Deaton et al., Bioorg. Med. Chem. Lett. 16: 978-983 (2006), or WO2006/012215, by reaction of an organic aldehyde with an amine as in equation 2: 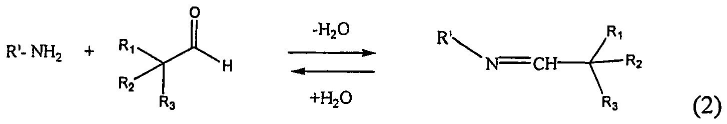 wherein each of R₁, R₂ and R₃ is independently selected from H, alkyl, substituted alkyl, cycloalkyl, substituted cycloalkyl, heterocyclyl, substituted heterocyclyl, allcylheterocyclyl, substituted alkylheterocyclyl, alkenyl, substituted alkenyl, aryl, substituted aryl, heteroaryl, substitute heteroaryl, alkylaryl, substituted alkylaryl, hydroxy, alkoxy, amino, alkylamino, mercapto, alkylmercapto, aryloxy, substituted aryloxy, heteroaryloxy, substituted heteroaryloxy, alkoxycarbonyl, acyl, acyloxy, acylamino, alkylsulfonyl, alkylsulfinyl, F, Cl, Br, NO₂ and cyano; or two or more of R₁, R₂ and R₃ are taken together to form a substituted or unsubstituted carbocyclic or heterocyclic ring structure; and

R' is selected from H, alkyl, substituted alkyl, cycloalkyl, substituted cycloalkyl, alkenyl, substituted alkenyl, aryl, substituted aryl, heteroaryl and substituted heteroaryl.

In other embodiments, the protected organic aldehyde is an iminium salt. Those skilled in the art recognize that such derivatives can be obtained in a variety of ways, such as, for example, by the methods described by Paukstelis et al., J. Org. Chem. 28:3021-3024 (1963), by reaction of an organic aldehyde with a secondary amine salt as in equation 3: 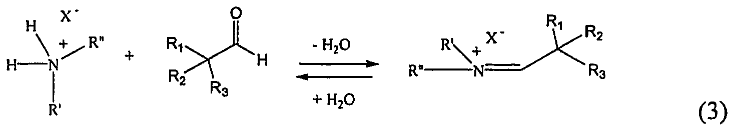 wherein each of R₁, R₂, R₃ and R' is as defined above with respect to equation 2;

R" is selected from H, alkyl, substituted alkyl, cycloalkyl, substituted cycloalkyl, alkenyl, substituted alkenyl, aryl, substituted aryl, heteroaryl and substituted heteroaryl.;
and X represents any suitable and pharmaceutically acceptable counter anion, such as chloride, bromide, phosphate, carbonate, sulfate, acetate or any other non-toxic, physiologically compatible anion.

In another embodiment, the protected organic aldehyde is a hydrazone. Those skilled in the art recognize that such derivatives are prepared in a number of ways such as, for example, by the methods disclosed in U.S. Patent Nos. 6,518,269 and 4,983,755, by reaction of an organic aldehyde with a hydrazine as in equation 4: 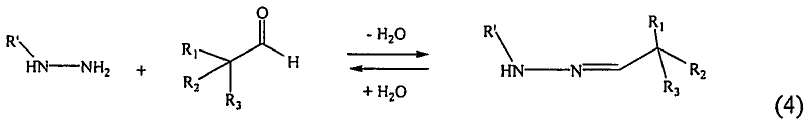 wherein each of R₁, R₂, R₃ and R' is as defined above with respect to equation 2.

In yet another embodiment, the protected organic aldehyde is a carbazone. Those skilled in the art recognize that such derivatives can be obtained in a variety of ways such as, for example, using methods described by Herrmann et al., Chem. Commun. 2965-2967 (2006) by reaction of an organic aldehyde with a hydrazide (or acyl hydrazine) as in equation 5: 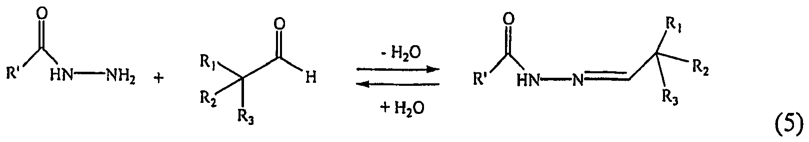 wherein each of R₁, R₂, R₃ and R' is as defined above with respect to equation 2.

In another embodiment, the protected organic aldehyde is a semicarbazone or thiosemicarbazone. Those skilled in the art recognize that such derivatives can be obtained in a variety of ways, such as, for example, using the methods described by Deaton et al., Bioorg. Med. Chem. Lett. 16:978-983 (2006) or by the methods disclosed in U.S. Patent No. 6,458,843, for example, by reaction of an organic aldehyde with a semicarbazine or thiosemicarbazine as in equation 6: 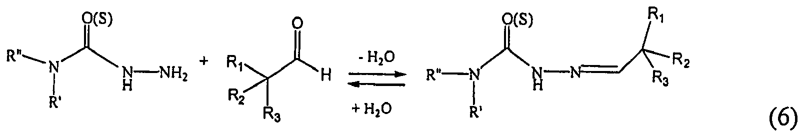 wherein each of R₁, R₂, R₃, R', R" is as defined above with respect to equations 2 and 3.

In still another embodiment, the protected organic aldehyde is an oxime. Those skilled in the art recognize that such derivatives can be obtained in a variety of ways, such as, for example, using the methods described by Reymond et al., Org. Biomol. Chem. 2:1471-1475 (2004) or U.S. Patent Application No. 2006/0058513, by reaction of an organic aldehyde with an oxoamine as in equation 7: 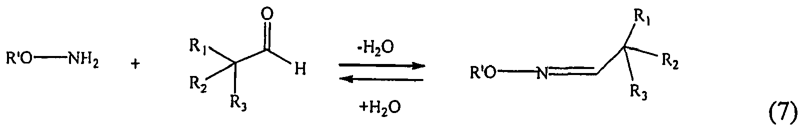 wherein each of R₁, R₂, R₃ and R' is as defined above with respect to equation 2.

In another embodiment, the protected organic aldehyde is an acetal or hemiacetal. Those skilled in the art recognize that such derivatives can be prepared in a variety of ways, such as, for example, by reaction of an aldehyde with one or more alcohols as in equation 8: 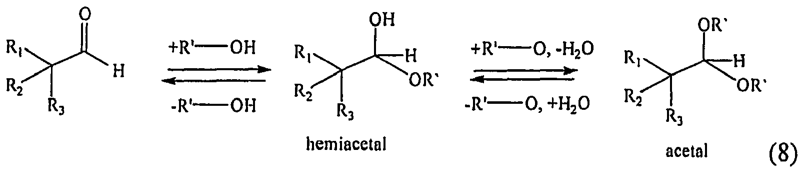 wherein each of R₁, R₂, R₃ and R' is as defined above with respect to equation 2.

In still another embodiment, the protected organic aldehyde is an α-hydroxy-1,3-dioxepane (or α-hydroxy-1,3-dioxane or α-hydroxy-1,3-dioxalane). Those skilled in the art recognize that such derivatives can be obtained in a variety of ways, such as, for example, by the methods disclosed in WO03/082850, by reaction of a hydroxy substituted organic aldehyde with another aldehyde, as in equation 9: 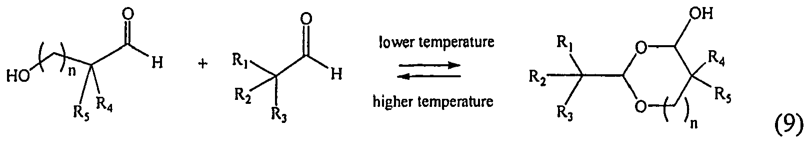 wherein each of R₁, R₂ and R₃ is as defined above with respect to equation 2; each of R₄ and R₅ is independently selected from H, alkyl, substituted alkyl, cycloalkyl, substituted cycloalkyl, heterocyclyl, substituted heterocyclyl, alkylheterocyclyl, substituted alkylheterocyclyl, alkenyl, substituted alkenyl, aryl, substituted aryl, heteroaryl, substituted heteroaryl, alkylaryl, substituted alkylaryl, hydroxy, alkoxy, amino, alkylamino, mercapto, alkylmercapto, aryloxy, substituted aryloxy, heteroaryloxy, substituted heteroaryloxy, alkoxycarbonyl, acyl, acyloxy, acylamino, alkylsulfonyl, alkylsulfinyl, F, Cl, Br, NO₂ and cyano; or R₄ and R₅ are taken together to form a substituted or unsubstituted carbocyclic or heterocyclic ring structure; and n is 1, 2 or 3.

The reaction shown in equation 9 is an energetically favorable cyclization (dimerization) that occurs spontaneously when the compounds are cooled together (1:1) to room temperature. When heated (*e.g*., to physiological temperatures), they separate again. Compound 4 is an example of a compound that forms a dimer upon cooling to room temperature.

In yet another embodiment, the protected organic aldehyde is an α-keto-1,3-dioxepane (or α-keto-1,3-dioxane, α-keto-1,3-dioxalane or α-keto-1,3-dioxetane). Those skilled in the art recognize that such derivatives can be obtained in a variety of ways, such as, for example, by the methods described by Xu et al., Tet. Lett., 46:3815-3818 (2005) or Krall et al., Tetrahedron 61:137-143 (2005), by reaction of an organic aldehyde with a hydroxy acid, thereby forming a protected aldehyde, as in equation 10: 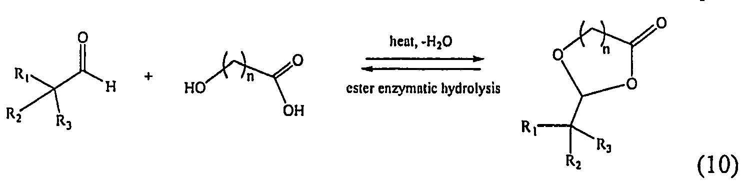 wherein each of R₁, R₂ and R₃ is as defined above with respect to equation 2; and n is 0, 1,2, or 3.

In another embodiment, the protected organic aldehyde is a macrocyclic ester/imine. Those skilled in the art recognize that such derivatives can be obtained in a variety of ways, such as, for example, as described in U.S. Patent No. 6,251,927, by reaction of a hydroxy substituted organic aldehyde with a compound of the formula HOOC-(CH₂)ₘ-NH₂, thereby forming a protected aldehyde, as in equation 11: 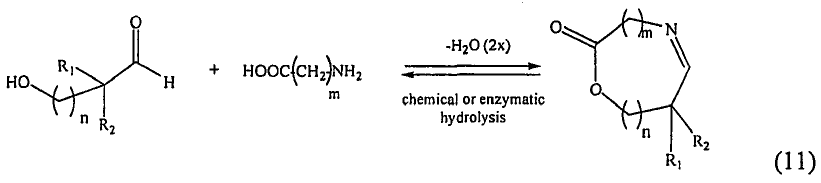 wherein R₁ and R₂ are as defined above with respect to equation 2; n is 0, 1, or 2; and m is 1 or 2.

Hydrolysis of the compound formed in equation 11 occurs by chemical hydrolysis through the imine, or enzymatic hydrolysis through the ester group.

In another embodiment, the protected organic aldehyde is a macrocyclic ester/hemiacetal. Those skilled in the art recognize that such derivatives can be obtained in a variety of ways, such as, for example, as described in U.S. Patent No. 6,251,927 by reaction of a hydroxy substituted organic aldehyde with a hydroxy acid having the structure HOOC-(CH₂)ₘ-OH, thereby forming a protected aldehyde, as in equation 12: HOOC-(CH₂)ₘ-OH, thereby forming a protected aldehyde, as in equation 12: 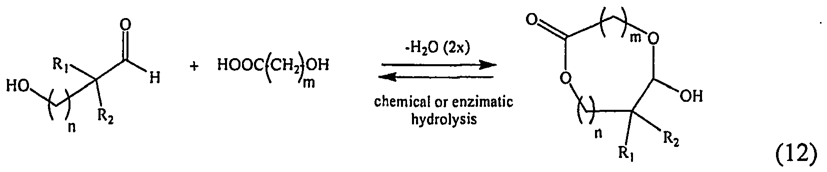 wherein R₁, R₂, m and n are as defined above with respect to equation 11.

Hydrolysis of the compound formed in equation 12 occurs by chemical hydrolysis through the ketal, or enzymatic hydrolysis through the ester group.

In still another embodiment, the protected organic aldehyde is a thiazolidine or a tetrahydro-1,3-thiazine. Those skilled in the art recognize that such derivatives can be obtained in a variety of ways, such as, for example, by employing the methods described by Jellum et al., Anal. Biochem. 31:339-347 (1969), Nagasawa et al., J. Biochem. Mol. Tox. 16:235-244 (2002), Roberts et al., Chem. Res. Toxicol. 11:1274-82 (1998) or U.S. Patent No. 5,385,922. Certain thiazolidines and tetrahydro-1,3-thiazines contemplated for use as described herein are represented by Formula III: 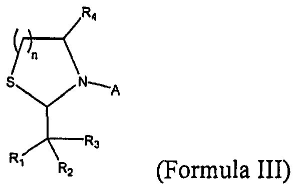 wherein each of R₁, R₂, R₃ and R₄ is independently selected from H, alkyl, substituted alkyl, cycloalkyl, substituted cycloalkyl, heterocyclyl, substituted heterocyclyl, alkylheterocyclyl, substituted alkylheterocyclyl, alkenyl, substituted alkenyl, aryl, substituted aryl, heteroaryl, substituted heteroaryl, alkylaryl, substituted alkylaryl, hydroxy, alkoxy, amino, alkylamino, mercapto, alkylmercapto, aryloxy, substituted aryloxy, heteroaryloxy, substituted heteroaryloxy, alkoxycarbonyl, acyl, acyloxy, acylamino, alkylsulfonyl, alkylsulfinyl, F, Cl, Br, NO₂, and cyano; or two or more of R₁, R₂ and R₃ are taken together to form a substituted or unsubstituted carbocyclic or heterocyclic ring structure;

A is selected from H, alkyl, substituted alkyl, cycloalkyl, substituted cycloalkyl, alkenyl, substituted alkenyl, aryl, substituted aryl, heteroaryl, substituted heteroaryl, alkylaryl, substituted alkylaryl, alkoxycarbonyl, acyl, acyloxy, acylamino, alkylsulfonyl and alkylsulfinyl; and n is 1 or 2.

In another embodiment, the protected organic aldehyde is an oxazolidine or a tetrahydro-1,3-oxazine. Those skilled in the art recognize that such derivatives can be obtained in a variety of ways, such as, for example, by employing the methods described by Bundgaard et al., Int. J. Pharma. Chem. 10:165-175 (1982), Sélambarom et al., Tetrahedron 58:9559-9556 (2002) or U.S. Patent No. 7,018,978. Certain oxazolidines and tetrahydro-1,3-oxazines contemplated for use as described herein are represented by Formula IV: 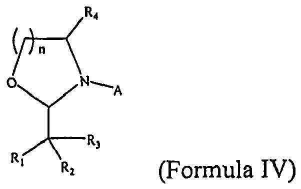 wherein each of R₁, R₂, R₃, R₄ and A and n is as described above with respect to formula III.

In still another embodiment, the protected organic aldehyde is an imidazolidine or a 1,3-hexahydro-pyrimidine. Those skilled in the art recognize that such derivatives can be obtained in a variety of ways, such as, for example, by employing the methods described by Lambert, J. Org. Chem. 52:68-71 (1987) or Fülöp, J. Org. Chem. 67:4734-4741 (2002). Certain imidazolidines and 1,3-hexahydro-pyrimidines contemplated for use as described herein are represented by Formula V: 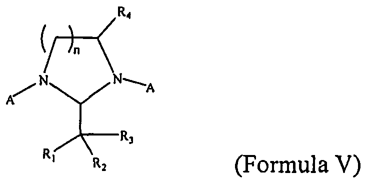 wherein each of R₁, R₂, R₃, R₄, n and A (selected independently at each occurrence) is as described above with respect to Formula III.

In yet another embodiment, the protected organic aldehyde is an imidazolidinone. Those skilled in the art recognize that such derivatives can be obtained in a variety of ways, such as, for example, by employing the methods described by Bundgaard et al., Int. J Parma. Chem. 23:163-173 (1985). Certain imidazolidinones contemplated for use as described herein are represented by Formula VI: 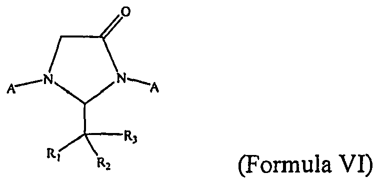 wherein each of R₁, R₂, R₃ and A (selected independently at each occurrence) is as described above with respect to Formula III.

In another embodiment, the protected organic aldehyde is an acyloxyalkyl ester or O-acyloxyalkyl derivative. Those skilled in the art recognize that such derivatives can be obtained in a variety of ways, such as, for example, by employing the methods described by Nudelman et al., Eur J. Med. J. Chem. 36: 63-74 (2001), Nudelman et al., J. Med. Chem. 48:1042-1054 (2005), or Swedish Patent No. SE9301115. Certain acyloxyalkyl esters contemplated for use as described herein are represented by Formula VII: 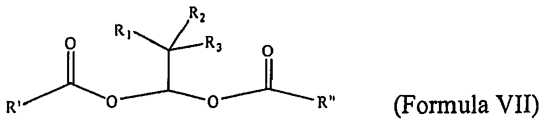 wherein each of R₁, R₂, and R₃ is as defined above with respect to Formula III, and each ofR' and R" is selected independently from H, alkyl, substituted alkyl, cycloalkyl, substituted cycloalkyl, alkenyl, substituted alkenyl, aryl, substituted aryl, heteroaryl, and substituted heteroaryl. In certain embodiments, in addition to releasing the active aldehyde upon metabolic hydrolysis *in vivo,* an acyloxyalkyl ester derivative also releases butyric acid. Butyric acid prodrugs have been reported to provide increased aqueous solubility and permeability across cell membranes (Nudelman et al., Eur J. Med. J. Chem. 36: 63-74 (2001)).

In another embodiment, the protected organic aldehyde is an N-acyloxyalkyl derivative. Those skilled in the art recognize that such derivatives can be obtained in a variety of ways, such as, for example, by employing the methods described by Bundgaard et al., Int. J. Pharm. 22:454-456 (1984) and Bundgaard et al., Int. J. Pharm. 13:89-98 (1983). Certain N-acyloxyalkyl derivatives contemplated for use as described herein are represented by Formula VIII: 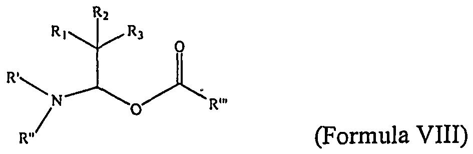 wherein each of R₁, R₂, R₃, R' and R" is as described above with respect to Formulas III and V; and R"' is selected from H, alkyl, substituted alkyl, cycloalkyl, substituted cycloalkyl, alkenyl, substituted alkenyl, aryl, substituted aryl, heteroaryl, and substituted heteroaryl.

In another embodiment, the protected organic aldehyde is the salt of an N-acyloxyalkyl derivative. Those skilled in the art recognize that such derivatives can be obtained in a variety of ways, such as, for example, by employing the methods described by Bodor et al., J. Med. Chem. 23:469-474 (1980) or U.S. Patent No. 3,998,815. The salts of N-acyloxyalkyl derivatives contemplated for use as described herein are represented by Formula IX: 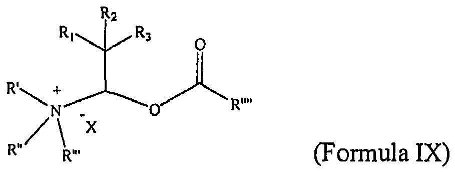 wherein each of R₁, R₂, R₃, R', R", and R"' is as defined above with respect to Formula VI;

R"" is selected from H, alkyl, substituted alkyl, cycloalkyl, substituted cycloalkyl, alkenyl, substituted alkenyl, aryl, substituted aryl, heteroaryl, and substituted heteroaryl; and

X represents a suitable and pharmaceutically acceptable counter anion, as described above with respect to equation 3.

In yet another embodiment, the protected organic aldehyde is a 5-oxazolidinone.

Those skilled in the art recognize that such derivatives can be obtained in a variety of ways, such as, for example, by employing the methods described by Bundgaard et al., Int. J. Pharma. Chem. 46:159-167 (1988) or Ishai-Ben, J. Am. Chem. Soc. 79:5736-38 (1957). Certain 5-oxazolidinones contemplated for use as described herein are represented by Formula X: 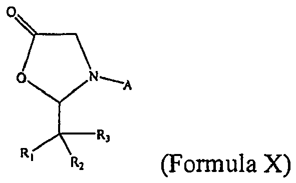 wherein each of R₁, R₂, R₃ and A is as defined above with respect to Formula III.

### Class 7- Encapsulated organic compounds that release CO either by an enzymatic process or by decarbonylation.

This system comprises the same molecules described under Class 6, but includes their encapsulation in host-guest supermolecules, liposomes, cyclodextrins, and other polymeric materials that are able to produce nanoencapsulated aggregattes.

Other sources of CO include: tricarbonyldichlororuthenium (II) dimmer, CORM-2 (Sigma) and tricarbonylchloro(glycinato)ruthenium (II), CORM-3 (Johnson, T. R. et al. Dalton Trans, 1500-8 (2007)).

The CO may be administered alone, in a pharmaceutical composition or combined with other therapeutic regimens. The CO and other therapeutic agent(s) may be administered simultaneously or sequentially. When the other therapeutic agents are administered simultaneously they can be administered in the same or separate formulations, but are administered at the same time. The other therapeutic agents may be administered sequentially with one another and with CO when the administration of the other therapeutic agents and the CO is temporally separated. The separation in time between the administration of these compounds may be a matter of minutes or it may be longer.

### Drug Formulations:

Compositions useful in the practice of this invention can be formulated as pharmaceutical compositions together with pharmaceutically acceptable carriers for parenteral administration or enteral administration of for topical or local administration. For example, the compositions useful in the practice of the invention can be administered as oral formulations in solid or liquid form, or as intravenous, intramuscular, subcutaneous, transdermal, or topical formulations. Oral formulations for local delivery are preferred.

The compositions are typically administered with pharmaceutically acceptable carriers. The term "pharmaceutically-acceptable carrier" as used herein means one or more compatible solid, or semi-solid or liquid fillers, diluants or encapsulating substances which are suitable for administration to a human or other mammal such as a dog, cat, horse, cow, sheep, or goat. The term "carrier" denotes an organic or inorganic ingredient, natural or synthetic, with which the active ingredient is combined to facilitate the application. The carriers are capable of being commingled with the preparations of the present invention, and with each other, in a manner such that there is no interaction which would substantially impair the desired pharmaceutical efficacy or stability. Carriers suitable for oral, subcutaneous, intravenous, intramuscular, etc. formulations can be found in Remington's Pharmaceutical Sciences, Mack Publishing Company, Easton, Pa.

Pharmaceutically acceptable carriers for oral administration include capsules, tablets, pills, powders, troches, and granules. In the case of solid dosage forms, the carrier can comprise at least one inert diluent such as sucrose, lactose or starch. Such carriers can also comprise, as is normal practice, additional substances other than diluents, e.g. lubricating agents such as magnesium stearate. In the case of capsules, tablets, troches and pills, the carrier can also comprise buffering agents. Carriers, such as tablets, pills and granules, can be prepared with coatings on the surfaces of the tablets, pills or granules which control the timing and/or the location of release of the pharmaceutical compositions in the gastrointestinal tract. In some embodiments, the carriers also target the active compositions to particular regions of the gastrointestinal tract and even hold the active ingredients at particular regions, such as is known in the art. Alternatively, the coated compounds can be pressed into tablets, pills, or granules. Pharmaceutically acceptable carriers include liquid dosage forms for oral administration, e.g. emulsions, solutions, suspensions, syrups and elixirs containing inert diluents commonly used in the art, such as water. Besides such inert diluents, compositions can also include adjuvants, such as wetting agents, emulsifying and suspending agents, and sweetening, flavoring agents.

The pharmaceutical preparations of the invention may be provided in particles. Particles as used herein means nano or microparticles (or in some instances larger) which can consist in whole or in part of the peripheral opioid antagonists or the other therapeutic agent(s) as described herein. The particles may contain the therapeutic agent(s) in a core surrounded by a coating, including, but not limited to, an enteric coating. The therapeutic agent(s) also may be dispersed throughout the particles. The therapeutic agent(s) also may be adsorbed into the particles. The particles may be of any order release kinetics, including zero order release, first order release, second order release, delayed release, sustained release, immediate release, and any combination thereof, etc. The particle may include, in addition to the therapeutic agent(s), any of those materials routinely used in the art of pharmacy and medicine, including, but not limited to, erodible, nonerodible, biodegradable, or nonbiodegradable material or combinations thereof. The particles may be microcapsules which contain the antagonist in a solution or in a semi-solid state. The particles may be of virtually any shape.

Both non-biodegradable and biodegradable polymeric materials can be used in the manufacture of particles for delivering the therapeutic agent(s). Such polymers may be natural or synthetic polymers. The polymer is selected based on the period of time over which release is desired. Bioadhesive polymers of particular interest include bioerodible hydrogels described by H.S. Sawhney, C.P. Pathak and J.A. Hubell in Macromolecules, (1993) 26:581-587, the teachings of which are incorporated herein. These include polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

Pharmaceutically acceptable carriers for intravenous administration include solutions containing pharmaceutically acceptable salts or sugars. Pharmaceutically acceptable carriers for intramuscular or subcutaneous injection include salts, oils, or sugars.

When used in its acid form, a compound of the present invention can be employed in the form of a pharmaceutically acceptable salt of the acid. Carriers such as solvents, water, buffers, alkanols, cyclodextrins and aralkanols can be used. Other auxiliary, non-toxic agents may be included, for example, polyethylene glycols or wetting agents.

The pharmaceutically acceptable carriers and compounds described in the present invention are formulated into unit dosage forms for administration to the patients. The dosage levels of active ingredients (i.e. compounds of the present invention) in the unit dosage may be varied so as to obtain an amount of active ingredient that is effective to achieve a therapeutic effect in accordance with the desired method of administration. The selected dosage level therefore mainly depends upon the nature of the active ingredient, the route of administration, and the desired duration of treatment. If desired, the unit dosage can be such that the daily requirement for an active compound is in one dose, or divided among multiple doses for administration, e.g. two to four times per day.

Preferably, the compounds are administered orally. The preferred dose levels will be determined in animals for representative compounds. All CORM compounds described in the present invention generate CO after administration to the body. Although CO is generated preferentially at the sites of the gastrointestinal side-effect, some of the CO generated will bind to hemoglobin in red blood cells. Thus, dose finding studies will initially be guided by measurement of carboxyhemoglobin (COHb) levels in the blood. Methods for the measurement of COHb levels in the blood are well known and are being used on a regular basis in diagnostic laboratories. In normal healthy humans COHb levels are about 0.5% in healthy nonsmokers and up to 9% in smokers. Preferred dose levels of the compounds described in the present invention are such that no significant rise in COHb levels is observed. However, in some applications a transient rise in COHb levels up to 10% may be tolerated. This level of COHb is not associated with any symptoms.

As representative examples, compounds in Classes 1 and 4 are administered in a dosage ranging between 5 and 25 mmol/day depending on the nature of the CO containing compound and its molar CO content. The same range of dosage of the CO containing molecule is applied for Class 3 compounds. For aspirin conjugates in classes 2 and 5, the dose can vary from a lower 120 mg/day up to 10 g day with preferred values in the range of 1 g/day for adults. These are indicative values dependent on the nature of the CO carrier molecular fragment and comply with the usual ranges for aspirin dosage. For the polyhalomethane and similar compounds in Class 6, e.g., dichloromethane, the dose range varies between 0.01 to 10 mmol/kg per os, with a preferred dose level of 0.1 mmol/kg. The same range of dosage of active principle is applied in the Class 7 compounds.

In general, dosage is adjusted appropriately to achieve desired drug levels, locally or systemically. In the event that the response in a subject is insufficient at such doses, even higher doses (or effective higher doses by a different, more localized delivery route) may be employed to the extent that patient tolerance permits.

The pharmaceutical preparations of the invention, when used in alone or together with NSAIDs in cocktails or as covalent conjugates, are administered in therapeutically effective amounts. A therapeutically effective amount will be that amount which establishes a level of the drug(s) effective for treating a subject, such as a human subject. An effective amount means that amount alone or with multiple doses, necessary to delay the onset of, inhibit completely or lessen the progression of or halt altogether the onset or progression of a gastrointestinal side-effect caused by NSAIDs or alcohol, and preferably, the side-effect known as gastric ulcer. When administered to a subject, effective amounts will depend, of course, on the particular side effect chosen as the endpoint; the severity of the condition; individual patient parameters including age, physical condition, size and weight; concurrent treatment; frequency of treatment; and the mode of administration. These factors are well known to those of ordinary skill in the art and can be addressed with no more than routine experimentation.

The CO is administered in conjunction with NSAID therapy. This means that the CO is administered close enough in time to the NSIAD therapy so as to inhibit the gastrointestinal side effects caused by the NSAID therapy. Typically, the CO will be administered at the same time as the NSAID therapy or on the same day as the NSAID therapy. It will be understood, however, that the CO may be administered prior to or subsequent to the NSAID therapy, such as one or two days before or one or two days after the NSAID therapy.

Also disclosed are medical products. The medical product includes a CORM containing vial and, optionally, an NSAID containing vial. The medical product also includes indicia indicating that the CORM is for inhibiting a gastrointestinal side-effect of NSAID therapy. The indicia can be on a label attached to the CORM containing vial or can be in a package containing the CORM containing vial.

The present invention discloses the ability of CO to protect the gastric mucosa against damage by NSAIDs. The discovery of this protective effect of CO forms the basis for the development of new, CO based treatment modalities that prevent gastric ulcer induction by NSAIDs. The preventive effect may be achieved by the oral application of liquid or solid formulations that are loaded with dissolved or entrapped CO or by oral administration of CORMs that release CO in the stomach. The examples below demonstrate that oral administration of a CORM (ALF 186 (Formula I)) elevates COHb levels in rats. An inactive form of ALF186, that does not release CO, failed to prevent ulcer induction by indomethacin, indicating that the ability of ALF 186 to prevent gastric ulcer induction by indomethacin is mediated by CO that is released from ALF186.

The CO and CORM patents and prior art in the literature describe the use of CO or CORMs for the treatment or prevention of diseases associated with inflammation and/or ischemia/reperfusion injury. Disease indications for CO related therapy of disorders of the gastrointestinal tract included inflammatory bowel disease (Hegazi, Rao et al. 2005) postoperative ileus (Moore, Otterbein et al. 2003), cardiovascular disease, renal disease and inflammation (Motterlini et al. 2005). They have also been used for the treatment of gastric ulcers caused by Helicobacter pylori (WO 2008/130261). Since the NSAID induced gastropathy is not an inflammatory disease, the use of CO has not previously been considered for this disease indication. The reasons why CO protects the gastric mucosa against indomethacin induced hemorrhagic lesions are not clear. CO has anticoagulant, anti-apoptotic and vasorelaxant effects, all of which could contribute to its ability to protect the stomach against indomethacin induced ulcers.

Only marginal protection was seen after intraperitoneal application of ALF186 given at doses that elevate COHb levels to peak values as high as those observed after oral administration of protective doses of ALF186. This finding may be related to the known fact that delivery of CO to the intestines via the systemic route (i.e. after CO inhalation) is very poor (Vreman, Wong et al. 2005). To be therapeutically effective CO preferably is locally administered to the stomach wall from the lumen of the stomach. Oral application of ALF186 is associated with a "spill over" of CO to the blood and probably an inhibition of the peristaltic activity of the stomach as a result of smooth muscle cell relaxation by CO. To be optimally useful for clinical applications, CO can be delivered to therapeutic targets in the stomach wall (in particular epithelial cells and endothelial cells) without adverse effects such as stomach dilation and COHb elevations above safe levels (~5% COHb).

It may be advantageous to use CORMs that release CO more slowly than ALF186 or CORMs that preferentially release CO at the low pH values of the gastric juice. A pH sensitive compound known as CORM-A1 has been described in the literature (Motterlini, Sawle et al. 2004) and in a patent (WO2005013691 A1). Alternatively, a stomach targeting strategy may be developed for CORMs that do not release CO but donate CO to intracellular targets. Many of the beneficial effects of CO are, at least in part, mediated by induction of HO-1 and thus endogenous CO production. Thus, a stomach targeting strategy may also be applied to HO-1 inducers, many of which are found in plant extracts. While the clinical use of HO-1 inducers has been disclosed in patents for a variety of disease indications (US60663334; WO9609038), HO-1 induction has not previously been considered for the prevention of NSAID induced ulcers. Coadministration of a CORM or of an HO-1 inducer with NSAIDs is an application of the present invention.

Another application is the establishment and use of CORM-NSAID conjugates in which a CO releasing moiety is linked via a digestible spacer to an NSAID. CORM-NSAID conjugates can be expected to have less gastrointestinal side effects than the parent NSAIDs. Since CO released from CORMs has anti-inflammatory effects, CORM-NSAID conjugates can also be expected to be as effective or even more effective than the parent NSAIDs in the treatment of inflammatory diseases. Indeed, ALF186, the CORM used in the experiments that led to the present invention, was as effective as indomethacin in reducing carrageenan induced paw edema in rats. Thus, some CORMs may be useful as anti-inflammatory drugs that are superior to NSAIDs in that they do not cause gastrointestinal side effects. However, optimal reduction of pain and inflammation may require the combined use of a CORM and an NSAID. Finally, daily administration of a CORM-aspirin conjugate could be used for cardioprotection without an elevated risk of harmful events in the gastrointestinal tract. CO, a product of the metabolism of the CORM-aspirin conjugates, may not only counteract the induction of gastrointestinal ulcers by aspirin, but may also complement the cardioprotective effect of aspirin by its anticoagulant and anti-inflammatory effects.

### EXAMPLES

The following examples involve the use of the CORM:

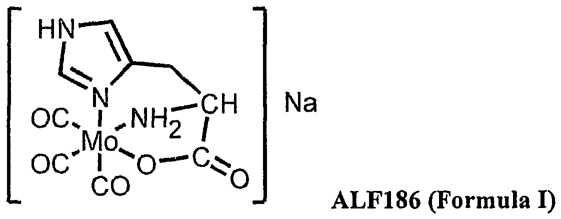

ALF 186 was prepared according to the original literature description in W. Beck, W. petri, J. Meder, J. Organomet. Chem., 191, 73 (1980).

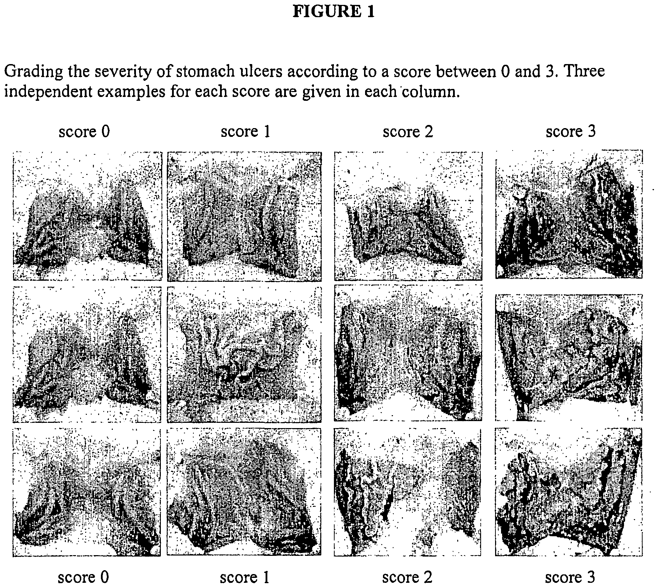

*Example 1:* Ulcers can be readily induced in rats or mice by the oral or subcutaneous application of ethanol or of an NSAID such as indomethacin. In rats ulcers are readily recognizable a few hours after indomethacin treatment as black streaks on the surface of the gastric mucosa. For a rough estimate of ulcer severity lesions may be scored as grade 0, 1, 2, and 3 (Fig 1). The hemorrhagic lesions that are visible as black streaks are caused by the death of epithelial and endothelial cells leading to bleeding ulcers and coagulation of blood on the surface of the mucosa. To test the effect of CO on gastric ulcer induction a molybdenum containing CORM, ALF186 (Formula I), was used. This compound spontaneously releases CO upon solution in aqueous media. Male Sprague Dawley rats were treated with ALF186 by the oral route (200 mg/kg) and the intraperitoneal route (50 mg/kg) and received 10 minutes later 1 ml of ethanol. The treatment with ALF186 almost completely prevented gastric ulcers that were induced by ethanol (Table 1).

**Table 1: Protection against ethanol induced ulcers by a CORM (ALF186) in rats**

| GROUP | n | ULCER INDUCTION | TREATMENT (mg/kg) | ULCER SEVERITY |
|---|---|---|---|---|
| 1 | 3 | Ethanol 1 ml oral | water | 3+, 3+, 3+ |
| 2 | 3 | Ethanol 1 ml oral | ALF186 (200 oral + 50 ip) | 1, 1, 1 |

In a separate experiment rats were treated with ALF186 and an inactive form of ALF186 referred to as iALF186. The inactive compound iALF186 had released all CO during incubation in water for 12 hours at room temperature. Protection against indomethacin induced lesions was observed in ALF186 treated rats, but not in iALF186 treated rats.

*Example 2:* Male Sprague Dawley rats that were deprived of food, but not water, for 18 hrs were injected subcutanously with indomethacin at doses of 30 or 80 mg/kg. Half of the animals received ALF 186 (300 mg/kg) by the oral route 5 minutes before the administration of indomethacin. Four and a half hours later the animals were sacrificed by CO2 inhalation. The results show that treatment with ALF 186 completely prevented the hemorrhagic lesions that were induced by either 30 or 80 mg/kg of indomethacin (table 2).

**Table 2: Protection against indomethacin induced ulcers by a CORM (ALF186) in rats**

| GROUP | n | ULCER INDUCTION (mg/kg) | TREATMENT (mg/kg) | ULCER SEVERITY |
|---|---|---|---|---|
| 1 | 3 | Indomethacin sc (30) | none | 1, 1, 1 |
| 2 | 3 | Indomethacin sc (80) | none | 1, 3, 3 |
| 3 | 3 | Indomethacin sc (30) | ALF186 oral (300) | 0, 0, 0 |
| 4 | 3 | Indomethacin sc (80) | ALF186 oral (300) | 0, 0, 0 |

In additional experiments protection was observed when ALF186 was administered immediately before indomethacin or 3 hrs before indomethacin, but not when administered 18 hrs before indomethacin. Protection against ulcer was associated with COHb levels above 30%.

*Example 3:* To test whether ALF186 interfered with the anti-inflammatory effect of indomethacin, male Sprague Dawley rats were deprived of food but not water for 18 hours and then treated with water (the vehicle used for ALF186), indomethacin (30 mg/kg, sc), ALF186 (300 mg/kg, oral), or both i.e. ALF186 and indomethacin. Half an hour later a local inflammation was induced in the right paws of all rats by intradermal injection of carragenan. Treatment with ALF186 not only prevented ulcer induction by indomethacin as previously observed, but also reduced paw edema as effectively as indomethacin (Table 3).

**Table 3: The CORM ALF186 does not interfere with the anti-inflammatory effect of indomethacin in rats.**

| GROUP | n | PAW INFLAMMATION (mg/kg) | TREATMENT (mg/kg) | RIGHT PAW VOLUME (ml) at 6 hrs | ULCER SEVERITY at 6 hrs |
|---|---|---|---|---|---|
| 1 | 3 | Carragenan id right paw | water | 2-22; 2.01; 2:08 | 0, 0, 0 |
| 2 | 3 | Carragenan id right paw | Indomethacin sc (30) | 1.91; 1.90; 1.61 | 2, 2, 1 |
| 3 | 3 | Carragenan id right paw | ALF166 oral (300) | 1.64; 1.56; 1.65 | 0, 0, 0 |
| 4 | 3 | Carragenan id right paw | Indomethacin sc (30) +ALF186 oral (300) | 1.75; 1.41; 1.81 | 0, 0, 0 |

This finding is consistent with the well known anti-inflammatory effects of CO. Since the combination of both drugs was as effective as each drug alone the possibility cannot be excluded that ALF186 had an effect on the action of indomethacin, although this is unlikely.

*Example 4:* To test whether systemic application of ALF186 by the i.p. route had any protective effect on indomethacin induced ulcers, male Sprague Dawley rats were deprived of food, but not water, and then treated with indomethacin and water or indomethacin and ALF186, given either by the oral route (100 mg/kg) or by the intraperitoneal route (45 or 15 mg/kg). These doses were chosen since previous experiments have shown that similar levels of COHb were generated by ALF186 given ip at a dose of 45 mg/kg and given by the oral route at a dose of 100 mg/kg. While oral dosing of ALF186 at 100 mg/kg completely prevented indomethacin induced ulcer induction, the intraperitoneal dosing of ALF186 at doses of 45 mg/kg or 15 mg/kg had no effect on ulcer incidence and severity (Table 4).

**Table 4: Systemic administration of a CORM (ALF186) is less effective than oral administration in protecting rats against indomethacin induced ulcers**

| GROUP | n | ULCER INDUCTION (mg/kg) | TREATMENT (mg/kg) | ULCER SEVERITY |
|---|---|---|---|---|
| 1 | 4 | Indomethacin sc (80) | ALF136 Ip (45) | 2, 1, 3, 3 |
| 2 | 4 | Indomethacin sc (80) | ALF186 Ip (15) | 3, 3, 3, 3 |
| 3 | 4 | Indomethacin sc (80) | ALF186 oral (100) | 0, 0, 0, 1 |
| 4 | 4 | Indomethacin sc (80) | water | 3, 3, 3, 3 |
| 5 | 2 | none | water | 0, 0 |

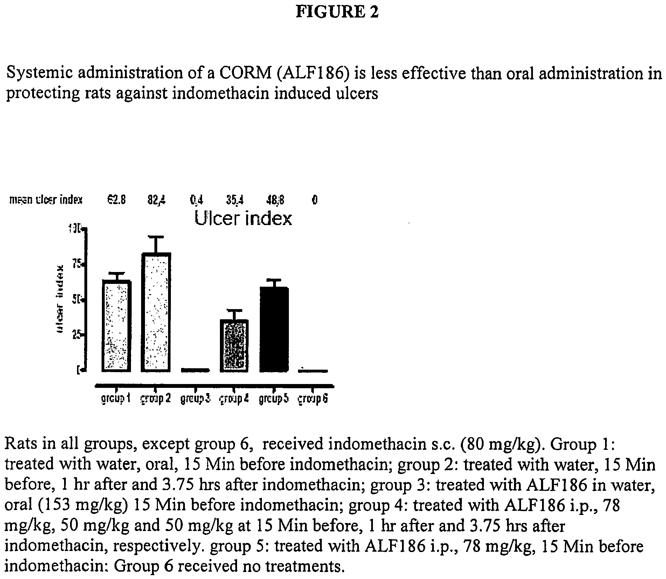

In a subsequent study, ALF186 was given by the oral route in a single dose, in a single dose i.p., or in three consecutive doses i.p.. Three injections of ALF186 given i.p. increased COHb levels to a similar extent than oral application of ALF186. While protection against ulcers was complete in rats treated with ALF186 by the oral route, the crude stage 0-3 scoring illustrated in Figure 1 did not reveal a clear protection in rats that received a single or multiple i.p. doses of ALF186. However when a more accurate ulcer index scoring method was used that is based on measurements of the lengths of lesions, a partial protection was seen in rats that received three consecutive i.p. injections of ALF 186 (Fig. 2).

*Example 5:* Male Sprague Dawley rats were deprived of food but not water and then treated by oral administration of ALF186 (100 mg/kg oral), water (vehicle for ALF186). Five minutes after the treatment with CORMs all rats received subcutaneous injections of indomethacin (80 mg/kg). Complete protection against indomethacin induced ulcers was observed in rats treated with ALF186 administered orally. A partial protection was observed in rats treated ip.

**Table 5: Systemic administration of a CORM (ALF186) is less effective than oral administration in protecting rats against indomethacin induced ulcers**

| GOUP | n | ULCER INDUCTION (mg/kg) | TREATMENT (mg/kg) | Time of administration relative to indo | ULCER SEVERITY | Sum |
|---|---|---|---|---|---|---|
| 1 | 5 | Indomethacin sc (80) | Water oral | -15 Min | 2; 2.5; 3; 3; 2 | 12.5 |
| 2 | 5 | Indomethacin sc (80) | Water ip | -15 Min, 1 hr, 3,75 hrs | 2.5; 3; 2; 2.5, 3 | 13 |
| 3 | 5 | Indomethacin sc (80) | ALF186 oral (153) | -15 Min | 0, 0, 0, 0, 0 | 0 |
| 4 | 5 | Indomethacin sc (80) | ALF186 ip (78+50+50) | -15 Min, 1 hr, 3,75 hrs | 1; 3; 3; 2; 2 | 11 |
| 5 | 5 | Indomethacin sc (80) | ALF 186 Ip (78) | -15 Min | 3, 2; 2, 3; 3 | 13 |
| 6 | 5 | none | none | | 0, 0, 0, 0, 0 | 0 |

The foregoing results are confirmed with other CORMs, in similar experiments, including a CORM in olive oil as a vehicle.

### REFERENCES

List of patents and references cited:
WO1995009831Del Soldato 1995: Nitric esters having anti-inflammatory and/or analgesic activity and process for their preparation
US5700947: Del Soldato 1997: Nitric esters having anti-inflammatory and/or analgesic activity and process for their preparation
US5621000: Arena and del Soldato 1997: Nitric esters having a pharmacological activity and process for their preparation
US5861426: Del Soldato 1999: Nitro compounds of the formula A-X1-NO2 and their compositions having anti-inflammatory, analgesic and anti-thrombotic activities
US6040341: Del Soldato and Sannicolo 2000: Compounds and their compositions having anti-inflammatory and anti-thrombotic activities
US6242432 B1: Del Soldato 2001: Antithrombotic organic nitrates)
WO2005013691 A1: Motterlini and Alberto, 2005. Use of boranocarbonates for the therapeutic delivery of carbon monoxide
US2003068387A1: Buelow and Woo: Carbon monoxide generating compounds for the treatment of vascular, inflammatory and immune disorders
WO 02/092075 A2: Motterlini and Mann: Therapeutic delivery of carbon monoxide WO20040445599: Motterlini and Mann: Therapeutic delivery of carbon monoxide
WO03066067A2 and US2004067261A1: Haas, Romao, Royo, Fernandes, Gonçalves: Method of treating a mammal by administration of a compound having the ability to release CO, compounds having the ability to release CO and oharmaceutical composition thereof
WO9422482: Disorbo and Reeves. Compositions including heme-containing proteins and methods relating thereto
WO03000114 A3: Bach, Tobiasch, Soares, Otterbein, and Gose, (2003). Carbon monoxide improves outcomes in tissue and organ transplants and suppresses apoptosis
US 2002/0155166 A1: Choi, Lee, and Otterbein (2002).
   Carbon monoxide as a biomarker and therapeutic agent
WO2004/043341 A2: Billiar, Choi, McCloskey, Otterbein, Zuckerbaum (2004). Treatment of hemorrhagic shock
US 2004/0052866 A1 and WO7096977 A2: Otterbein, Choi, Zuckerbaum (2004) Methods of treating hepatius.
WO03/072024 A2 and US2003/0219496 A1: Otterbein, Choi, Bach, Zuckerbaum (2003). Methods of treating vascular disease.
WO003103585 A2: Otterbein, Choi, (2003) Methods of treating angiogenesis, tumor growth, and metastasis.
WO 2008/130261 : Nobre, Seixas, Romao, Saraiva (2008) Treatment of Infections by carbon monoxide Bandarage, U. K. and D. R. Janero (2001). "Nitric oxide-releasing nonsteroidal antiinflammatory drugs: novel gastrointestinal-sparing drugs." Mini Rev Med Chem 1(1): 57-70.
Birnboim, H. C. and H. Privora (2000). "Depletion of intracellular glutathione reduces mutations by nitric oxide-donating drugs." Nitric Oxide 4(5): 496-504.
de Leval, X., F. Julemont, et al. (2004). "First and second generations of COX-2 selective inhibitors." Mini Rev Med Chem 4(6): 597-601.
Dorward, S., A. Sreedharan, et al. (2006). "Proton pump inhibitor treatment initiated prior to endoscopic diagnosis in upper gastrointestinal bleeding." Cochrane Database Syst Rev(4): CD005415.
Ekstrom, P., L. Carling, et al. (1996). "Prevention of peptic ulcer and dyspeptic symptoms with omeprazole in patients receiving continuous non-steroidal antiinflammatory drug therapy. A Nordic multicentre study." Scand J Gastroenterol 31(8): 753-8.
Elliott, S. N. and J. L. Wallace (1998). "Nitric oxide: a regulator of mucosal defense and injury." J Gastroenterol 33(6): 792-803.
Fiorucci, S. and E. Antonelli (2006). "NO-NSAIDs: from inflammatory mediators to clinical readouts." Inflamm Allergy Drug Targets 5(2): 121-31.
Fiorucci, S., L. Santucci, et al. (2003). "Gastrointestinal safety of NO-aspirin (NCX-4016) in healthy human volunteers: a proof of concept endoscopic study." Gastroenterology 124(3): 600-7.
Flower, R. J. (2003). "The development of COX2 inhibitors." Nat Rev Drug Discov 2(3): 179-91.
Goldstein, J. L. (2004). "Challenges in managing NSAID-associated gastrointestinal tract injury." Digestion 69 Suppl 1: 25-33.
Grosser, N. and H. Schroder (2000). "A common pathway for nitric oxide release from NO-aspirin and glyceryl trinitrate." Biochem Biophys Res Commun 274(1): 255-8.
Hawkey, C. J. (2000). "Nonsteroidal anti-inflammatory drug gastropathy." Gastroenterology 119(2): 521-35.
Hegazi, R. A., K. N. Rao, et al. (2005). "Carbon monoxide ameliorates chronic murine colitis through a heme oxygenase 1-dependent pathway." J Exp Med 202(12): 1703-13.
Hooper, L., T. J. Brown, et al. (2004). "The effectiveness of five strategies for the prevention of gastrointestinal toxicity induced by non-steroidal anti-inflammatory drugs: systematic review." Bmj 329(7472): 948.
Howard, P. A. and P. Delafontaine (2004). "Nonsteroidal anti-Inflammatory drugs and cardiovascular risk." J Am Coll Cardiol 43(4): 519-25.
Jacobs, E. J., M. J. Thun, et al. (2007). "A large cohort study of long-term daily use of adult-strength aspirin and cancer incidence." J Natl Cancer Inst 99(8): 608-15.
Jones, M. K., H. Wang, et al. (1999). "Inhibition of angiogenesis by nonsteroidal antiinflammatory drugs: insight into mechanisms and implications for cancer growth and ulcer healing." Nat Med 5(12): 1418-23.
Juni, P., L. Nartey, et al. (2004). "Risk of cardiovascular events and rofecoxib: cumulative meta-analysis." Lancet 364(9450): 2021-9.
Keeble, J. E. and P. K. Moore (2002). "Pharmacology and potential therapeutic applications of nitric oxide-releasing non-steroidal anti-inflammatory and related nitric oxide-donating drugs." Br J Pharmacol 137(3): 295-310.
Laine, L. (1996). "Nonsteroidal anti-inflammatory drug gastropathy." Gastrointest Endosc Clin N Am 6(3): 489-504.
Maines, M. D. (1997). "The heme oxygenase system: a regulator of second messenger gases." Annu Rev Pharmacol Toxicol 37: 517-54.
McGeer, P. L. (2000). "Cyclo-oxygenase-2 inhibitors: rationale and therapeutic potential for Alzheimer's disease." Drugs Aging 17(1): 1-11.
McMurray, R. W. and K. J. Hardy (2002). "Cox-2 inhibitors: today and tomorrow." Am J Med Sci 323(4): 181-9.
Miller, M. R. and I. L. Megson (2007). "Recent developments in nitric oxide donor drugs." Br J Pharmacol 151(3): 305-21.
Minamiyama, Y., S. Takemura, et al. (2007). "Cytochrome P450 is responsible for nitric oxide generation from NO-aspirin and other organic nitrates." Drug Metab Pharmacokinet 22(1): 15-9.
Moore, B. A., L. E. Otterbein, et al. (2003). "Inhaled carbon monoxide suppresses the development of postoperative ileus in the murine small intestine." Gastroenterology 124(2): 377-91.
Motterlini, R., B. E. Mann, et al. (2003). "Bioactivity and pharmacological actions of carbon monoxide-releasing molecules." Curr Pharm Des 9(30): 2525-39.
Motterlini, R., P. Sawle, et al. (2004). "CORM-A1: a new pharmacologically active carbon monoxide-releasing molecule." Faseb J.
Motterlini, R. et al. (2005). "Therapeutic applications of carbon monoxidereleasing molecules." Expert Opinion on Investigational Drugs 14(11): 1305-1318
Needleman, P. and P. C. Isakson (1997). "The discovery and function of COX-2." J Rheumatol Suppl 49: 6-8.
Prescott, S. M. and F. A. Fitzpatrick (2000). "Cyclooxygenase-2 and carcinogenesis." Biochim Biophys Acta 1470(2): M69-78.
Rahme, E. and H. Nedjar (2007). "Risks and benefits of COX-2 inhibitors vs nonselective NSAIDs: does their cardiovascular risk exceed their gastrointestinal benefit? A retrospective cohort study." Rheumatology (Oxford) 46(3): 435-8.
Raskin, J. B., R. H. White, et al. (1995). "Misoprostol dosage in the prevention of nonsteroidal anti-inflammatory drug-induced gastric and duodenal ulcers: a comparison of three regimens." Ann Intern Med 123(5): 344-50.
Rostom A., C. Dube, et al. (2002). "Prevention of NSAID-induced gastroduodenal ulcers." Cochrane Database Syst Rev(4): CD002296.
Rostom, A., K. Muir, et al. (2007). "Gastrointestinal Safety of Cyclooxygenase-2 Inhibitors: A Cochrane Collaboration Systematic Review." Clin Gastroenterol Hepatol.
Ryter, S. W. and L. E. Otterbein (2004). "Carbon monoxide in biology and medicine." Bioessays 26(3): 270-80.
Salinas, G., U. C. Rangasetty, et al. (2007). "The Cycloxygenase 2 (COX-2) Story: It's Time to Explain, Not Inflame." J Cardiovasc Pharmacol Ther 12(2): 98-111.
Silverstein, F. E. (1998). "Improving the gastrointestinal safety of NSAIDs: the development of misoprostol--from hypothesis to clinical practice." Dig Dis Sci 43(3): 447-58.
Topol, E. J. (2004). "Failing the public health--rofecoxib, Merck, and the FDA." N Engl J Med 351(17): 1707-9.
Vreman, H. J., R. J. Wong, et al. (2005). "Determination of carbon monoxide (CO) in rodent tissue: effect of heme administration and environmental CO exposure." Anal Biochem 341(2): 280-9.
Wilder-Smith, C. H., B. Jonzon, et al. (2006). "Dose-effect comparisons of the CINOD AZD3582 and naproxen on upper gastrointestinal tract mucosal injury in healthy subjects." Scand J Gastroenterol 41(3): 264-73.
Wolfe, M. M., D. R. Lichtenstein, et al. (1999). "Gastrointestinal toxicity of nonsteroidal antiinflammatory drugs." N Engl J Med 340(24): 1888-99.

## Claims

1. CO for use in a method of inhibiting NSAID or alcohol induced gastric ulcer, the method comprising:
(a) administering to a subject in need of such inhibition an effective amount of said CO; or
(b) instructing a subject undergoing NSAID therapy to take an effective amount of CO for the purpose of inhibiting NSAID or alcohol induced gastric ulcer; or
(c) providing the subject with a package containing a CORM, and
providing the subject with indicia indicating that the CORM is for inhibiting NSAID or alcohol induced gastric ulcer.

2. CO for use according to claim 1, wherein the subject is undergoing NSAID therapy for inflammation, pain, fever, a thromboembolic disorder, patent ductus arteriosus, Bartter's syndrome, cancer, a condition associated with pathological angiogenesis, psoriasis, Alzheimer's disease, and/or shock,
or wherein the subject is undergoing NSAID therapy for a condition other than an inflammatory condition.

3. CO for use according to claim 1, wherein the CO is administered as a gas, or wherein the CO is administered as a CORM.

4. CO for use according to claim 3, wherein the CORM is a CO containing organometallic complex or wherein the CORM is an organic compound.

5. CO for use according to claim 3, wherein the CORM is formulated in a pharmaceutically acceptable carrier that is an alginate solution, or wherein the CORM is formulated with a carrier that binds to the mucosal surface in the stomach and/or the duodenum.

6. CO for use according to claim 3 wherein the CO is administered as a CORM, and wherein the method further comprises:
(a) administering an NSAID to the subject; or
(b) instructing the subject to take an NSAID; or
(c) providing the subject with an NSAID.

7. CO for use according to claim 6, wherein said NSAID is admixed with the CORM, or wherein said NSAID is covalently conjugated to the CORM via a digestible linker.

8. CO for use according to any one of claims 1-7, wherein the CO is administered orally.

9. The use of a CORM in the manufacture of a medicament for the treatment of NSAID or alcohol induced gastric ulcer.

10. The use of claim 9, wherein the CORM is a CO containing organometallic complex or wherein the CORM is an organic compound.

## Patentansprüche

1. CO zur Verwendung in einem Verfahren zur Hemmung von durch NSAID oder Alkohol ausgelösten Magengeschwüren, wobei das Verfahren Folgendes umfasst:
(a) Verabreichen einer wirksamen Menge des CO an ein Individuum, das einer solchen Hemmung bedarf; oder
(b) Anleiten eines Individuums, das eine NSAID-Therapie durchläuft, eine wirksame Menge CO einzunehmen, um durch NSAID oder Alkohol ausgelöstes Magengeschwür zu hemmen; oder
(c) Versorgen des Individuums mit einer Verpackung, die ein CORM enthält, und Versorgen des Individuums mit Informationen, dass das CORM zur Hemmung von durch NSAID oder Alkohol ausgelösten Magengeschwüren dient.

2. CO zur Verwendung nach Anspruch 1, wobei das Individuum eine NSAID-Therapie gegen Entzündung, Schmerz, Fieber, ein Thromboembolieleiden, persistierenden Ductus arteriosus, Bartter-Syndrom, Krebs, ein Leiden in Verbindung mit pathologischer Angiogenese, Psoriasis, Alzheimer-Krankheit und/oder Schock durchläuft oder wobei das Individuum eine NSAID-Therapie gegen ein anderes Leiden als ein Entzündungsleiden durchläuft.

3. CO zur Verwendung nach Anspruch 1, wobei das CO als Gas verabreicht wird oder wobei das CO als CORM verabreicht wird.

4. CO zur Verwendung als Anspruch 3, wobei das CORM ein CO-hältiger organometallischer Komplex ist oder wobei das CORM eine organische Verbindung ist.

5. CO zur Verwendung nach Anspruch 3, wobei das CORM in einem pharmazeutisch annehmbaren Träger formuliert ist, der eine Alginatlösung ist, oder wobei das CORM mit einem Träger formuliert ist, der an die Schleimhautoberfläche im Magen und/oder im Duodenum bindet.

6. CO zur Verwendung nach Anspruch 3, wobei das CO als CORM verabreicht wird und wobei das Verfahren weiters Folgendes umfasst:
(a) Verabreichen eines NSAID an das Individuum; oder
(b) Anweisen des Individuums, ein NSAID einzunehmen; oder
(c) Versorgen des Individuums mit einem NSAID.

7. CO zur Verwendung nach Anspruch 6, wobei das NSAID mit dem CORM vermischt wird oder wobei das NSAID über einen verdaulichen Linker kovalent an das CORM konjugiert ist.

8. CO zur Verwendung nach einem der Ansprüche 1-7, wobei das CO oral verabreicht wird.

9. Verwendung eines CORM zur Herstellung eines Medikaments zur Behandlung von durch NSAID oder Alkohol ausgelösten Magengeschwüren.

10. Verwendung nach Anspruch 9, wobei das CORM ein CO-hältiger organometallischer Komplex ist oder wobei das CORM eine organische Verbindung ist.

## Revendications

1. CO pour utilisation dans une méthode d'inhibition de AINS ou ulcère gastrique induit par l'alcool, la méthode comprenant:
(a) administrer à un sujet, qui a besoin d'une telle inhibition, une quantité efficace dudit CO; ou
(b) ordonner à un sujet sous thérapie AINS de prendre une quantité efficace de CO dans le but d'inhiber AINS ou l'ulcère gastrique induit par l'alcool; ou
(c) fournir au sujet un emballage contenant un CORM,
et
fournir au sujet des indices indiquant que le CORM est destiné à inhiber AINS ou l'ulcère gastrique induit par l'alcool.

2. CO pour utilisation selon la revendication 1, où le sujet subit la thérapie AINS pour l'inflammation, la douleur, la fièvre, un trouble thrombo-embolique, une persistance du canal artériel, le syndrome de Barter, un cancer, un état associé à l'angiogenèse pathologique, le psoriasis, la maladie d'Alzheimer et/ou un choc, ou bien où le sujet subit une thérapie AINS pour un état autre qu'un état inflammatoire.

3. CO pour utilisation selon la revendication 1, où le CO est administré comme un gaz, ou bien où le CO est administré comme un CORM.

4. CO pour utilisation selon la revendication 3, où le CORM est un complexe organométallique contenant CO, ou bien où le CORM est un composé organique.

5. CO pour utilisation selon la revendication 3, où le CORM est formulé dans un support pharmaceutiquement acceptable qui est une solution d'alginate, ou bien où le CORM est formulé avec un support qui se lie à la surface de la muqueuse dans l'estomac et/ou le duodénum.

6. CO pour utilisation selon la revendication 3, où le CO est administré comme CORM, et où la méthode comprend en outre:
(a) administrer un AINS au sujet; ou
(b) ordonner au sujet de prendre un AINS; ou
(c) fournir au sujet un AINS.

7. CO pour utilisation selon la revendication 6, où ledit AINS est mélangé avec le CORM, ou bien où ledit AINS est conjugué de manière covalente au CORM par un lieur digestible.

8. CO pour utilisation selon l'une quelconque des revendications 1 à 7, où le CO est administré oralement.

9. Utilisation d'un CORM dans la fabrication d'un médicament pour le traitement de AINS ou d'un ulcère gastrique induit par l'alcool.

10. Utilisation selon la revendication 9, où le CORM est un CO contenant un complexe organométallique ou bien où le CORM est un composé organique.
